Europäisches Patentamt

European Patent Office

Office européen des brevets

(19)

(11) **EP 1 512 012 B1**

(12) **EUROPEAN PATENT SPECIFICATION**

(45) Date of publication and mention
of the grant of the patent:
**25.10.2006 Bulletin 2006/43**

(21) Application number: **03740171.8**

(22) Date of filing: **02.06.2003**

(51) Int Cl.:
*G01N 33/557* (2006.01)   *G01N 33/558* (2006.01)
*G01N 33/551* (2006.01)   *G01N 33/68* (2006.01)

(86) International application number:
**PCT/EP2003/005748**

(87) International publication number:
**WO 2003/102585 (11.12.2003 Gazette 2003/50)**

(54) **BIOMOLECULAR KINETICS METHOD USING A FLOW-THROUGH MICROARRAY**

BIOMOLEKULARES KINETIK-MESSVERFAHREN MIT HILFE VON EINEM DURCHFLUSS-
MIKROARRAY

NOUVEAU PROCEDE POUR SURVEILLER DES INTERACTIONS BIOMOLECULAIRES

(84) Designated Contracting States:
**AT BE BG CH CY CZ DE DK EE ES FI FR GB GR
HU IE IT LI LU MC NL PT RO SE SI SK TR**

(30) Priority: **03.06.2002 EP 02447107**

(43) Date of publication of application:
**09.03.2005 Bulletin 2005/10**

(73) Proprietor: **PamGene B.V.**
**5211 RX  Den Bosch (NL)**

(72) Inventors:
• **VAN BEUNINGEN, Marinus, Gerardus, Johannus
NL-5345 RR Oss (NL)**
• **RUIJTENBEEK, Robby
NL-3524 CK Utrecht (NL)**

(74) Representative: **De Clercq, Ann G. Y. et al
De Clercq, Brants & Partners c.v.,
Edgard Gevaertdreef 10a
9830 Sint-Martens-Latem (BE)**

(56) References cited:
WO-A-00/31304      WO-A-97/25619
WO-A-99/02266      GB-A- 2 261 283
US-A- 5 726 010

• **SAPSFORD K E ET AL: "Kinetics of antigen
binding to arrays of antibodies in different sized
spots" ANAL. CHEM.;ANALYTICAL CHEMISTRY
NOV 15 2001, vol. 73, no. 22, 15 November 2001
(2001-11-15), pages 5518-5524, XP002263066**
• **STILLMAN B.A. ET AL ANALYTICAL
BIOCHEMISTRY vol. 295, 2001, pages 149 - 157**

EP 1 512 012 B1

SEQUENCE LISTING

<110> PamGene B.V.

<120> NOVEL METHOD FOR MONITORING BIOMOLECULAR INTERACTIONS

<130> PAM-006-PCT

<150> EP 02447107.0
<151> 2002-06-03

<160> 4

<170> PatentIn version 3.1

<210> 1
<211> 13
<212> PRT
<213> Artificial Sequence

<220>
<223> Peptide

<400> 1

Gly Cys Ala Ser Leu Gln Gly Met Asp Thr Cys Gly Lys
1               5                   10

<210> 2
<211> 14
<212> PRT
<213> Artificial Sequence

<220>
<223> Peptide

<400> 2

```
Ala Pro Asp Pro Phe Lys Gln Gly Val Asp Thr Cys Gly Lys
1               5                   10
```

<210> 3
<211> 12
<212> PRT
<213> Artificial Sequence

<220>
<223> Peptide

<400> 3

```
Cys Ala Phe Lys Gln Gly Val Asp Thr Cys Gly Lys
1               5                   10
```

<210> 4
<211> 16
<212> PRT
<213> Artificial Sequence

<220>
<223> Peptide

<400> 4

```
Gly Cys Ala Pro Asp Pro Phe Lys Gln Gly Val Asp Thr Cys Gly Lys
1               5                   10                  15
```

## Description

### Field of the Invention

The present invention relates to the field of molecule binding interaction studies. The present invention relates to the multi-dimensional, high-speed analysis of a large number of mixed molecular species or bio-molecules, commonly called "libraries". More particularly, the present invention relates to a novel method for monitoring binding interactions between arrayed bio-molecules and sample target-molecules. Further, the present invention relates to a novel method to study interaction events between bio-molecules under varying reaction conditions.

### Background

Model systems which mimic receptor binding and other cell-based assays are of increasing importance in molecular biology and pharma industry. Phenomena such as transporter activity, pre- and post-synaptic cell interactions, chemotaxic response, enzyme-ligand binding and the like are of relevance to pharmacology, clinical chemistry and basic research. Major development areas at pharmaceutical companies are targeted to find the most effective molecule interactions which, most often, comprises a high-affinity of a molecule for its target defining a high binding constant $K_{on}$ and a low dissociation constant $K_{diss}$.

Accordingly, the importance of pharmacokinetic models remains undisputed and generated pharmacokinetic remains indispensable for, among other applications, (1) predicting plasma, tissue, and urine drug levels with any dosage regimen; (2) calculating the optimum dosage regimen for an individual patient; (3) estimating the possible accumulation of drugs and/or metabolites; (4) correlating drug concentrations with pharmacologic and toxicologic activity (i.e. pharmacodynamics); evaluating differences in the rate or extent of acailability between formulations (i.e. bioequivalence); (6) describing how changes in physiology or disease affect the absorption, distribution, and/or elimination of the drug; (7) explaining drug-drug and food-drug interactions, and (8) characterization of promising new drug candidates. Pharmacokinetic data is deemed essential for predicting the behavior and fate of the drug candidate within the human body.

Typically, collections of molecules or "libraries" are prepared and screened for molecules having a specified bioactivity, as indicated initially by detection of binding between one or more bio-molecules in the library and a target-molecule with which it reacts to, e.g., influence some biological process. Typically, libraries consist of a complex assortment of molecules containing one or more bio-molecules which may bind to a target of interest. The identification of bio-molecules which bind may provide a lead for identifying compounds with a desired biological activity, e.g. as a potential drug candidate.

As methods have become available to screen these complex mixtures or libraries more effectively, interest in exploiting this approach even increases.

The recent prior art discloses various methods for implementing the search for novel agents such as, for example, pharmacological or therapeutic agents (i.e. drug discovery), agents useful in animal and human care or management, agriculturally useful chemicals, selective biocides for insects, weeds, or other pests, and catalytic and other entities useful in industrial chemical, bio-chemical and pharmaceutical processes.

Monoclonal antibody technology has markedly changed modem chemical and medical analytical techniques. Antibody based detection systems such as ELISA (Enzyme Linked ImmunoSorbent Assays) and radioimmunoassays have been developed to take advantage of antibody specificity and sensitivity. However, while sensitive, these techniques are time-consumlng and' usually require multiple manipulations and/or reagent additions.

Methods of identifying analyte-molecules to a target have been described earlier, e.g. US 5,861,254, which discloses a flow cell method for the analysis of nucleic acid molecular interaction kinetics. A disadvantage of these so-called biosensor chips is the low capacity of the system of different target species and the high sample volumes needed. High throughput systems allowing for the screening of arrays of target-molecules include e.g. WO 02/18648 which discloses a method for the analysis of binding interactions, thereby providing compound interaction site profiles; or US 5,324,633 which discloses a method and apparatus for measuring the binding affinity of a receptor to a ligand by using an array of immobilized polymers. Although said known high throughput systems have yet been recognized for their major contribution in lowering sample volumes. Stillman and Tonkinson (2001, Analytical Biochemistry 295, 149-157) disclose experiments for studying the binding conditions between an immobilized target and a sample analyte in function of time under varying lengths of the immobilized species and substrate types. A disadvantage, however, is that the described methods require the initiation of a new experiment for each varying binding condition being studied, in this case varying the length of the immobilized species or varying the substrate. The experimental time needed, reproducibility and sensitivity are still subject to improvement and continuous efforts in those fields are well appreciated in the art.

Accordingly, it is an object of the present invention to provide a high-throughput method for high-speed monitoring of target/analyte interactions with a much improved sensitivity level.

## Summary of the Invention

A novel method for qualitatively and quantitatively evaluating the interaction of a target/analyte complex is provided by virtue of the present invention.
The present invention is directed to a method for assaying target-molecules such as drug candidates. More specifically, the present invention provides a novel method for monitoring and measuring the binding interaction between at least one analyte and an array of immobilized target-molecules to determine binding interaction parameters and pharmacokinetic parameters of said molecules.

The aforementioned and additional objects of the invention are accomplished by a method for monitoring interactions between target-molecules and at least one analyte comprising:

(a) contacting a plurality of target-molecules, said target-molecules being immobilized on a porous substrate with a sample, said sample comprising at least one analyte;
(b) incubating said target-molecules with said sample under conditions supporting target-molecule/analyte interaction ;
(c) monitoring said interaction as defined in step (b), said monitoring being in function of time of incubation as defined in step (b);
(d) optionally determining at least one interaction parameter;
(e) varying said conditions as defined in step (b) by varying a reaction parameter, wherein said reaction parameter is chosen from the group comprising temperature, flow speed, pH and ionic strength; and;
(f) repeating, at least once, steps (c) to (e).

Said method according to the present specification allows for high-sensitive analysis of target/analyte interactions. More in particular, a method according to the present invention allows for kinetic analysis of an analyte in a sample present down to attomoles concentration.

Additional features and advantages of the invention will be set forth in the detailed description which follows, and in part will be apparent from the description, or may be learned by practice of the invention. The objectives and other advantages of the invention will be realized and attained by the process particularly pointed out in the written description and appended claims.

## Detailed Description of the Invention

The invention relates to methods and corresponding arrays especially suited to study binding kinetics of target/analyte interactions.

In the present specification and the appended claims, the singular forms "a", "an", and "the" include plural references unless the context clearly dictates otherwise. Unless defined otherwise, all technical and scientific terms used herein have the same meaning as commonly understood to one of ordinary skill in the art.

The present invention relates to methods and corresponding arrays for high-throughput binding interaction and pharmacokinetic studies of interaction events between target-molecules and analytes.

The terms "analyte" and "analyte molecule" are used interchangeable throughout the present invention. The term "analyte in a sample" refers to a molecule in a sample, i.e. a molecule to be analysed. Typically, analyte concentrations present in a sample for analysis according to a method as described in the present invention range from $5 \times 10^{-4}$ nM to 50 nM. Usually, analyte concentrations present in a sample for analysis according to a method as described in the present invention range from $5 \times 10^{-4}$ nM to 10 nM. More usually, analyte concentrations in a sample range from $1 \times 10^{-4}$ nM to 5 nM. More usually, analyte concentrations in a sample range from $5 \times 10^{-3}$ nM to 1 nM. More usually, analyte concentrations in a sample range from $1 \times 10^{-3}$ nM to $1 \times 10^{-1}$ nM. More usually, analyte concentrations in a sample range from $5 \times 10^{-2}$ nM to $1 \times 10^{-1}$ nM. More usually, analyte concentrations in a sample range from $5 \times 10^{-2}$ nM to $5 \times 10^{-1}$ nM.

Accordingly, one embodiment of the present invention provides a method as described herein, wherein said analyte is present in said sample in a concentration from $5 \times 10^{-4}$ nM to 10 nM.

In a further embodiment, said concentration ranges from $5 \times 10^{-3}$ nM to 1 nM.

In yet a further embodiment, said concentration ranges from $5 \times 10^{-2}$ nM to $10^{-1}$ nM.

An analyte as used in the present specification refers to any molecule which may associate or bind to a target-molecule immobilized onto a porous substrate for the purpose of performing micro-array analysis. The term analyte as used in the present specification refers both to separate molecules and to portions of molecules such as e.g. an epitope of a protein. Examples of analytes which may be employed in the present invention include, but are not limited to, antibodies including monoclonal antibodies polyclonal antibodies, purified antibodies, synthetic antibodies, antisera reactive with specific antigenic determinants (such as viruses, cells or other materials), proteins, peptides, polypeptides, enzyme binding sites, cell membrane receptors, lipids, proteolipids, drugs, polynucleotides, oligonucleotides, sugars, polysaccharides, cells, cellular membranes and organelles, nucleic acids including deoxyribonucleic acids (DNA), ribonucleic acids (RNA), and peptide nucleic acids (PNA) or any combination thereof; cofactors, lectins, metabolites, enzyme substrates, metal ions and metal chelates.

Accordingly, the present invention relates to a method as described herein, wherein said analyte is selected from the group comprising antibodies, monoclonal antibodies, antibody fragments, antisera, polynucleotides, nucleic acids, peptides, enzyme binding sites, polysaccharides, cells, cellular membranes, and organelles.

The analytes may be naturally-occurring or man-made molecules. Also, they may be employed in their unaltered state or as aggregates with other species. Analytes may contain modifications, both naturally occurring or induced *in vitro* or *in vivo.* Induced modifications include adduct formation such as hapten attachment, multimerization, complex formation by interaction with other chemical moieties, digestion or cleavage (by, for example, protease), and metal ion attachment or removal. Analytes may be attached, covalently or non-covalently to, to a binding member, either directly or via a mediating linker.

Specific examples of analyte/target interactions which may be investigated by the present invention include but are not limited to the hereunder mentioned ligand/receptor interactions as well-known in the art:

(a) micro-organism receptors: determination of ligands which bind to receptors, such as specific transport proteins or enzymes essential to survival of micro-organisms, is useful, as in the discovery of a new class of antibiotics. Of particular value may be antibiotics against opportunistic fungi, protozoa, and those bacteria resistant to the antibiotics in current use;

(b) enzymes: for instance, determination of the binding site of enzymes such as the enzymes responsible for cleaving neurotransmitters is useful. Also of value may be determination of ligands which bind to certain receptors to modulate the action of the enzymes which cleave the different neurotransmitters is useful in the development of drugs which may be used in the treatment of disorders of neurotransmission;

(c) antibodies: for instance, the present invention may be useful in investigating the ligand-binding site on the antibody molecule which combines with the epitope of an antigen of interest; determining a sequence that mimics an antigenic epitope may lead to the development of vaccines in which the immunogen is based on one or more such sequences or may lead to the development of related diagnostic agents or compounds useful in therapeutic treatments such as for auto-immune diseases (e.g. by blocking the binding of the "self' antibodies). For purposes of the present invention, antibody includes a whole antibody or an antibody fragment (Fab or (Fab)$_2$);

(d) catalytic polypeptides: polymers, preferably polypeptides, which are capable of promoting a chemical reaction involving the conversion of one or more reactants to one or more products. Such polypeptides generally include a binding site specific for at least one reactant or reaction intermediate and an active functionality proximate to the binding site, which functionality is capable of chemically modifying the bound reactant;

(e) hormone receptors: for instance, the receptors for insulin and growth hormone. Determination of the ligands which bind with high affinity to a receptor is useful in the development of, for example, an oral replacement of the daily injections which diabetics must take to relieve the symptoms of diabetes, and in a replacement for the scarce human growth hormone which may only be obtained from cadavers or by recombinant DNA technology. Other examples are vaso-constrictive hormone receptors; determination of those ligands which bind to a receptor may lead to the development of drugs to control blood pressure;

(f) opiate receptors: determination of ligands which bind to the opiate receptors in the brain is useful in the development of less-addictive replacements for morphine and related drugs.

Virtually any sample may be analyzed using the method according to the present specification. However, usually, the sample is a biological or a biochemical sample. The term "biological sample," as used herein, refers to a sample obtained from an organism or from components (e.g., cells) of an organism. The sample may be of any biological tissue or fluid. Frequently the sample will be a "clinical sample" which is a sample derived from a patient. Such samples include, but are not limited to, sputum, cerebrospinal fluid, blood, blood fractions such as serum including fetal serum (e.g., SFC) and plasma, blood cells (e.g., white cells), tissue or fine needle biopsy samples, urine, peritoneal fluid, and pleural fluid, or cells there from. Biological samples may also include sections of tissues such as frozen sections taken for histological purposes.

Examples of biochemical samples include, without limitation, cell line cultures, purified functional protein solutions, polypeptide solutions, nucleic acid solutions including oligonucleotide solutions, and others.

Samples may be analyzed directly or they may be subject to some preparation prior to use in the assays of this invention. Non-limiting examples of said preparation include suspension/dilution of the sample in water or an appropriate buffer or removal of cellular debris, e.g. by centrifugation, or selection of particular fractions of the sample before analysis. Nucleic acid samples, for example, are typically isolated prior to assay and, in some embodiments, subjected to procedures, such as reverse transcription and/or amplification (e.g., polymerase chain reaction, PCR) to increase the concentration of all sample nucleic acids (e.g., using random primers) or of specific types of nucleic acids (e.g., using polynucleotide-thymidylate to amplify messenger RNA or gene-specific primers to amplify specific gene sequences). The amplification method set out in WO 99/43850 may also be used in the present invention.

The terms "target" and "target-molecule" are used interchangeable throughout the present invention and refer to molecules immobilized on a substrate. A wide variety of different molecules can be immobilized on the substrate of the present arrays. Similarly, the present methods are applicable to a wide variety of different molecules or targets that may be immobilized on the substrate of the present arrays. A target or target-molecule as used in the present specification refers to any molecule which may be attached to a substrate for the purpose of performing microarray analysis. A target further refers to a molecule that may be recognized by and/or interact with a particular analyte.

The methods and arrays are particularly exemplified herein in terms of antibodies as targets immobilized on a substrate, but they are equally applicable to other types of molecules. For example, one of skilled in the art could easily adapt the present methods and arrays to apply to targets including, but not limited to, agonists and antagonists for cell membrane receptors; toxins and venoms; viral epitopes; hormones (e.g. steroids etc.) and hormone receptors; peptides; enzymes; drugs; lectins; sugars; carbohydrate binding proteins and other interactants; oligonucleotides; nucleic acids including deoxyribonucleic acids (DNA), ribonucleic acids (RNA), and peptide nucleic acids (PNA) or any combination thereof; oligosaccharides; proteins and protein interactants such as e.g. avidin and its derivatives; antibodies including monoclonal antibodies, polyclonal antibodies, synthetic antibodies, purified antibodies and crude (serum) antibodies; antibody fragments such as e.g. Fab fragments; antibody interaction agents such as protein A; aptamers; and small chemical entities, such as enzyme substrates, cofactors, metal ions, metal chelates, and haptens.

Accordingly, the present invention relates to a method as described herein, wherein said target-molecules are selected from the group comprising hormone receptors, peptides, enzymes, oligonucleotides, nucleic acids, oligosaccharides, proteins, monoclonal antibodies, antibody fragments, haptens, and aptamers.

The methods according to the present invention are useful in the study of the kinetics associated with the dynamic processes of absorption, distribution, metabolism, and excretion (ADME) of a drug and/or its metabolites within a living organism. "Absorption" refers to the process of uptake of a drug compound from the site of administration into the systemic circulation. The transfer of drugs across the intestinal lumen is generally referred to as oral absorption, whereas the transfer of drugs across an external physiological barrier is referred to general absorption. A pharmacokinetic parameter of absorption may be estimated from kinetic data associated with a microarray having, for example, a plurality of appropriate liposomes immobilized thereon.

"Distribution" refers to the transfer of a drug compound from the site of administration to the total systemic circulation and then to extracellular and intracellular water and tissues. Drug distribution is usually a rapid and reversible process. A pharmacokinetic parameter of distribution may be estimated from kinetic data associated with a microarray having, for example, a plurality of appropriate plasma proteins, liposomes, and/or transport proteins immobilized thereon.

"Metabolism" refers to the sum of all the chemical reactions for bio-transformation of endogenous and exogenous substances which take place in the living cell. A pharmacokinetic parameter of metabolism may be estimated from kinetic data associated with a microarray having, for example, a plurality of appropriate metabolic enzymes immobilized thereon.

"Excretion" refers to the final elimination or loss of a drug from the body. Drug excretion includes both passive diffusion and relative specific carrier mediated excretion. Drugs may be excreted, unchanged or as metabolites, in urine via the kidneys or in feces via the bile and/or the intestine. Volatile compounds are often excreted in expired air by the lungs. As disclosed herein, the pharmacokinetic parameter of excretion may be estimated from kinetic data associated with a microarray having immobilized thereon, for example, an antibody that specifically detects the drug, as well as other proteins/receptors having a high affinity/specificity against the drug candidate. Such antibodies and proteins/receptors may be used to quantify the concentration/amount of the drug in different body fluids (e.g., urine/feces) and tissues, using a direct binding assay.

Accordingly, target-molecules immobilized to the substrate may include liposomes, plasma proteins, CYP 450 enzymes, other metabolic enzymes, and/or transport efflux proteins.

Interactions that may be monitored by the methods of the present invention include, but are not limited to, immunoassays; ligand-(membrane)receptor interactions; antigen-antibody interactions; protein-protein interactions; enzyme-substrate interactions; RNA-protein interactions; DNA-protein interactions; DNA-DNA interactions; cell-protein interactions; and drug screening. Further, an estimate of an absorption parameter of a drug candidate may be determined from the binding interactions between the drug candidate and one or more appropriate liposomes; an estimate of a distribution parameter of a drug candidate may be determined from the binding interactions between the drug candidate and an appropriate set of plasma proteins (e.g., specific and non-specific tissue binding and tissue permeability); an estimate of a metabolism parameter of a drug candidate may be determined from the binding interactions between the drug candidate and an appropriate set of metabolic enzymes; and an estimate of an excretion parameter of a drug candidate may be determined from the binding interactions between the drug candidate and an appropriate set of transport proteins.

Said interaction events may be monitored by the detection of signals generated as a result of said interaction.

Accordingly, in one embodiment of the present invention, a method is provided wherein said monitoring comprises the steps of :

(a) detecting a signal, said signal resulting from a target-molecule/analyte interaction, and;
(b) recording the level of said signal as defined in step (a), said level being indicative for the strength of target-molecule/analyte interaction.

Thereto, usually, the samples are processed so that the analyte molecule(s) of interest are labeled with a detectable label. The system may contain more labels producing different signals which may be a component of, or released by, an interaction event. Any combination of labels, e.g. first and second labels, first, second, and third labels, etc. may be employed for analyte sets, provided the labels are distinguishable from one another. Examples of distinguishable labels are well-known in the art and include: two or more different wavelength fluorescent dyes, such as Cy3 and Cy5 or Alexa 542 and Bodipy 630/650; two or more isotopes with different energy of emission, such as $^{32}P$ and $^{33}P$; labels which generate signals under different treatment conditions, like temperature, pH treatment by additional chemical agents, etc.; and labels which generate signals at different time points after treatment.

Using one or more enzymes for signal generation allows for the use of an even greater variety of distinguishable labels based on different substrate specificity of enzymes (e.g. alkaline phosphatase/peroxidase).

The term "label" as used in this specification refers to a molecule propagating a signal to aid in detection and quantification. Said signal may be detected either visually (e.g., because it has color, or generates a colored product, or emits fluorescence) or by use of a detector that detects properties of the reporter molecule (e.g., radioactivity, magnetic field, etc.). In the present specification, labels allow for the detection of the interaction or association of two molecules. Detectable labels suitable for use in the present invention include but are not limited to any composition detectable by spectroscopic, photochemical, biochemical, immunochemical, electrical, optical or chemical means.

Useful labels in the present invention include for instance biotin for staining with labeled streptavidin conjugate, digoxigenin, anti-digoxigenin, luciferase, P-galactosidase, antigens, enzymes and enzyme conjugates, (e.g. horseradish peroxidase, alkaline phosphatase and others commonly used in e.g. ELISA).

Magnetic beads (e.g. Dynabeads TM), colored glass or plastic (e.g. polystyrene, polypropylene, latex, etc.) beads, isotopic labels including radiolabels (e.g. $^3H$, $^{125}I$, $^{35}S$, $^{14}C$, $^{33}P$ or $^{32}P$), colorimetric labels such as colloidal gold (e.g. gold particles in the 40 nm to 80 nm diameter size range scatter green light with high efficiency), sugars, and lectins may also be useful. Patents teaching the use of such labels include U.S. Patent Nos. 3,817,837; 3,850,752; 3,939,350; 3,996,345; 4,277,437; 4,275,149; and 4,366,241. Molecules that change their fluorescence or activity upon a change in binding, may also be useful.

Particular suitable labels that may be employed in the invention may be chromogens including those that absorb light in a distinctive range of wavelengths so that a color may be observed or, alternatively, that emit light when irradiated with radiation of a particular wavelength or wavelength range, e.g., fluorescent molecules. Particular useful fluorescent labels include, by way of example and not limitation, fluorescein isothiocyanate (FITC), rhodamine, malachite green, Oregon green, Texas Red, Congo red, SybrGreen, phycoerythrin, allophycocyanin, 6-carboxyfluorescein (6-FAM), 2', 7'-dimethoxy-4',5'-dichloro-6-carboxyfluorescein (JOE), 6-carboxy X-rhodamine (ROX), 6-carboxy-2',4',7',4,7-hexachlorofluorescein (HEX), 5-carboxyfluorescein (5-FAM), N,N,N',N'-tetramethyl-6-carboxyrhodamine (TAMRA), cyanine dyes (e.g. Cy5, Cy3), BODIPY dyes (e.g. BODIPY 630/650, Alexa542, etc), green fluorescent protein (GFP), blue fluorescent protein (BFP), yellow fluorescent protein (YFP), red fluorescent protein (RFP), and the like, (see, e.g., Molecular Probes, Eugene, Oregon, USA).

Means for detecting such labels are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with an enzyme substrate and detecting the reaction product produced by the action of the enzyme on the substrate, and colorimetric labels are detected by simply visualizing the colored label.

Fluorescent labels are particularly suitable because they provide very strong signals with low background. Fluorescent labels are also optically detectable at high resolution and sensitivity through a quick scanning procedure. Fluorescent labels offer the additional advantage that irradiation of a fluorescent label with light can produce a plurality of emissions. Thus, a single label can provide for a plurality of measurable events.

Accordingly, in one embodiment of the present invention, a method is provided, wherein the detectable signal is a light emitted signal.

As will be appreciated by a person skilled in the art, detectable signals may be the appearance, disappearance or changes of a detectable signal.

In a further embodiment of the present invention, a method is provided wherein the light emitted signal is a fluorescent signal.

In yet a further embodiment of the present invention, a method is provided wherein a fluorescent signal is emitted by a labeled analyte, said analyte being associated with a target-molecule.

Desirably, fluorescent labels should absorb light above about 300 nm, usually above about 350 nm, and more usually above about 400 nm, usually emitting at wavelengths greater than about 10 nm higher than the wavelength of the light absorbed.

It should be noted that the absorption and emission characteristics of a bound light emitting label may differ from the unbound label. It is therefore noted that the various wavelength ranges and characteristics of said labels indicate the labels as employed and not the label that is un-conjugated and characterized in an arbitrary solvent. It will be recognized that fluorescent labels are not to be limited to single species organic molecules, but may include inorganic molecules, multi-molecular mixtures of organic and/or inorganic molecules, crystals, hetero-polymers, and the like.

Detectable signal may equally be provided by chemiluminescent and bioluminescent labels. Chemiluminescent sources include compounds which becomes electronically excited by a chemical reaction and can then emit light which serves as the detectable signal or donates energy to a fluorescent acceptor. Alternatively, luciferins can be used in conjunction with luciferase or lucigenins to provide bioluminescence.

Analyte molecule(s) may be labeled before, during, or after the sample contacts the immobilized target-molecules by means of any method known in the art. So-called "direct labels" are detectable labels that are directly attached to or incorporated into the analyte molecule prior to interaction or binding with the cognate interaction or binding partner.

"Indirect labels" are attached to a component capable of binding to the analyte or -to a member of a binding pair, the other member of which is attached to the analyte molecule. In indirect labeling, the labeled component may be linked to the analyte before, during or after the analyte containing sample contacts the immobilized target-molecules. Thus, for example, the analyte molecule(s) may be biotinylated and then bound to the cognate target-molecule binding partner. After binding, an avidin-conjugated fluorophore may bind the biotin-bearing analyte molecule, providing a label that is easily detected. Labels may be attached to the analyte molecule(s) directly or through a linker moiety. In general, the site of label or linker-label attachment is not limited to any specific position. For example, in nucleic acid labeling, a label may be attached to a nucleoside, nucleotide, or analogue thereof at any position that does not interfere with detection or hybridization as desired.

Alternatively, a detectable signal for monitoring an interaction event according to a method as described in the present invention may be a refractive index, a cellular activity, an absorbance, a color shift, a fluorescence resonance energy transfer, a radioactive emission, a change in pH, a change in temperature, or a change in mass.

Accordingly, a method is provided for monitoring interactions between target-molecules and at least one analyte according to the present invention, wherein said detectable signal is selected from the group comprising a refractive index, a cellular activity, a light emission including fluorescence, a change in absorbance, a change in fluorescence, an absorbance, a color shift, a fluorescence resonance energy transfer, a radioactive emission, a change in pH, a change in temperature, and a change in mass.

Signal detection may be a quantitative or a qualitative observation or both. From the strength of the detected signals, the strength of the interaction events can be deduced and an interaction parameter may be calculated. Said interaction parameter may be in function of the amount of interaction between an immobilized target-molecule and an analyte.

Accordingly, the present invention relates to a method as described herein, wherein said interaction parameter may be a binding, binding affinity, relative affinity, apparent affinity, association constant, dissociation constant, logarithm of an affinity, free energy for binding, an absorption parameter, a distribution parameter, a metabolism parameter, or an excretion parameter of the analyte for an immobilized target-molecule.

More specifically, and in one embodiment of the present invention, said interaction parameter is chosen from the group comprising specificity, affinity, association constant, absorption parameter, distribution parameter, metabolism parameter and excretion parameter.

The term "binding affinity" as used in the present invention refers to the tendency of a target-molecule to associate with an analyte. Said association may be permanent or transient and is typically characterized by a change in one or more physical and/or chemical properties of one or both of the associating molecules. Target and analyte molecules that have an affinity for each other may specifically bind to each other. Binding affinity as used herein refers to the strength of the sum of total of non-covalent interactions between two molecules.

Measurement of binding affinity or calculation of binding affinity as used in the present specification refers to a measurement or calculation of the association constant for a reaction Target-molecule + Analyte $\rightarrow$ Target-molecule/ Analyte complex.

The term "association constant" as used in the present specification refers to the rate constant for association of a target with an analyte. As will be recognized by a person known in the art, the association constant for a typical reaction (Target-molecule + Analyte → Target-molecule/Analyte complex) is usually designated as $k_{ass}$ or $k_{on}$ or $k_1$ and is expressed as $M^{-1}sec^{-1}$.

The term "dissociation constant" as used in the present specification refers to the rate or "off-rate" for dissociation of a target-molecule from the analyte to which it associates and is an indication of the strength of binding between two molecules in terms of how easy it is to separate the complex. As will be recognized by a person skilled in the art, the dissociation constant for a typical reaction is usually designated as $k_{diss}$ or $k_{off}$ or $k_{-1}$ or $k_2$ and is and is expressed as $sec^{-1}$.

The nature and geometry of the porous substrate to be used in the present invention will depend upon a variety of factors, including, among others, the type of array (e.g., one-dimensional, two-dimensional or three-dimensional) and the mode of attachment (e.g., covalent or non-covalent). Generally, the substrate according to the present invention may be composed of any porous material which will permit immobilization of a target-molecule and which will not melt or otherwise substantially degrade under the reaction conditions used. In addition, where covalent immobilization is contemplated, the substrate should be polyfunctional or be capable of being polyfunctionalized or activated with reactive groups capable of forming a covalent bond with the target to be immobilized (e.g. carboxylic acids, aldehydes, amino groups, cyano groups, ethylenic groups, hydroxyl groups, inercapto groups and the like).

Functional groups which may be present on the surface and used for linking will be at least bifunctional, i.e. they will have one functional group or moiety capable of forming a linkage with the activated substrate and any other functional group or moiety capable of forming a linkage with another linker molecule or the target-molecule. Linkers may be useful in the application of spacing the target-molecules away from the substrate. Linkers may be long or short, flexible, semi-rigid or rigid, charged or uncharged, hydrophobic or hydrophilic, depending on the particular application.

In certain circumstances, linkers may be used to "convert" one functional group into another. For example, an amino-activated substrate can be converted into a hydroxyl-activated substrate by reaction with, for example, 3-hydroxy-propionic acid. In this way, substrate materials which cannot be readily activated with a specified reactive functional group can be conveniently converted into an appropriately activated substrate. Chemistries and reagents suitable for "converting" such reactive groups are well-known, and will be apparent to those having skill in the art.

Linkers may also be used, where necessary, to increase or "amplify" the number of reactive groups on the activated substrate. For this embodiment, the linker will have three or more functional groups. Following attachment to the activated substrate by way of one of the functional groups, the remaining two or more groups are available for attachment of polynucleotides. Amplifying the number of functional groups on the activated substrate in this manner is particularly convenient when the substrate cannot be readily activated with a sufficient number of reactive groups.

Suitable linkers or cross-linker molecules include, by way of example and not limitation, polypeptides such as polyproline or polyalanine, saturated or unsaturated bifunctional hydrocarbons such as 1-amino-hexanoic acid, polymers such as polyethylene glycol, etc., 1,4-Dimethoxytrityl-polyethylene glycol phosphoramidites useful for forming phosphodiester linkages with hydroxyl groups and are described, for example in Zhang *et al.,* 1991, Nucl. 20 Acids Res. 19: 3929-3933 and Durand *et al.*, 1990, Nucl. Acids Res. 18:6353-6359. Other useful linkers are commercially available.

The method described in WO 01/12846 can also be used in combination with the present invention.

A number of materials suitable for use as substrates in the present invention have been described in the art. Particular suitable materials for use as substrates in the present invention include any type of porous substrates known in the art. More particular suitable materials for use as substrates in the present invention include any type of solid porous substrates known in the art.

The substrate may be in the form of beads, particles, sheets, films or membranes and may be permeable. For example, the substrate may consist of bead or particles (such as conventional solid phase synthesis supports), fibers (such as glass wool or other glass or plastic fibers), glass or plastic capillary tubes, or metal oxide membranes. The porous substrate may be planar or have simple or complex shape. The surface to which the molecule is adhered may be an external surface or an internal surface of the porous substrate. Particularly where the surface is porous, the molecule is likely to be attached to an internal surface. Where the solid surface is porous, various pore sizes may be employed depending upon the nature of the system.

The material of the porous substrate may be, for example, a metal, a ceramic metal oxide or an organic polymer. In view of strength and rigidity, a metal or a ceramic metal oxide may be used. Above all, in view of heat resistance and chemicals resistance, a metal oxide may be used. In addition, metal oxides provide a substrate having both a high channel density and a high porosity, allowing high density arrays comprising different first binding substances per unit of the surface for sample application. In addition, metal oxides are highly transparent for visible light. Metal oxides are relatively cheap substrates that do not require the use of any typical microfabrication technology and, that offers an improved control over the liquid distribution over the surface of the support, such as electrochemically manufactured metal oxide membrane. Metal oxide membranes having through-going, oriented channels can be manufactured through electrochemical etching of a metal sheet.

Accordingly, in one embodiment of the present invention, a method is provided as described herein, wherein said porous substrate is a metal oxide substrate.

The kind of metal oxide is not especially limited, but can be preferably used. As a metal, for example, a porous substrate of stainless steel (sintered metal) can be used. For applications not requiring heat resistance, a porous substrate of an organic polymer can also be used if it is rigid.

Metal oxides considered are, among others, oxides of zirconium, silicium, mullite, cordierite, titanium, zeolite or zeolite analog, tantalum, and aluminum, as well as alloys of two or more metal oxides and doped metal oxides and alloys containing metal oxides.

In one embodiment, a method as described herein is provided, wherein said porous substrate is an aluminum oxide support.

The metal oxide membranes are transparent, especially if wet, which allows for assays using various optical techniques. Such membranes have oriented through-going channels with well-controlled diameter and useful chemical surface properties. Patent application WO 99/02266 is exemplary in this respect, and is specifically incorporated in the present invention. This application demonstrates the use of Anopore™ which is a preferred substrate according to the present invention.

In a further embodiment, a method as described herein is provided, wherein said porous substrate is a flow-through substrate.

A particular useful substrate has long branched or partially branched capillaries, which are interconnected inside the substrate. These so-called interconnections allow for more precisely determining kinetic binding parameters which allow for an improved view on interaction behavior in natural environments. Typically, the responses obtained by known methods reflect rather "isolated" interaction events, i.e. "one-on-one" interactions are being studied, and might not allow for extrapolation towards expected responses under natural reaction conditions.

Useful substrate thickness according to the invention may for instance range from 50 $\mu$m to 150 $\mu$m. A particular suitable example of substrate thickness is 60 $\mu$m. Particularly suitable substrate pore sizes are about 200 nm. These dimensions are not to be construed as limiting the present invention.

The porous nature of the substrate facilitates the pressurized movement of fluid, e.g. the sample solution, through its structure. In contrast to two dimensional substrates, the flow-through nature of a 3-dimensional substrate or microarray, as employed in the methods as described herein, gives significantly reduced hybridization times and increased signal and signal-to-noise ratios. Further, a positive or negative pressure may be applied to the arrays in order to pump the sample solution dynamically up and down through the substrate pores. This may enhance the diffusion of analyte to the target-molecules, may increase the rate of accumulation of analyte specifically bound to a binding target partner and accordingly may result in increased opportunity for rare high-affinity analyte molecules to bind to a target. Further, bound analyte molecules may be replaced by higher affinity analyte molecules over an extended period of exposure to the target, thus allowing for selection of those analyte molecules with the highest affinity for a given target.

Accordingly, in one embodiment of the present invention, a method as described herein is provided, wherein said incubating of target-molecules and sample is dynamic.

In a further embodiment, said dynamic incubation comprises subjecting said porous substrate to at least one cycle of forward and backward flow of sample across said porous substrate.

In yet a further embodiment, said forward and backward flow is established by application of alternating positive and negative pressure.

The duration of one cycle of forward and backward flow of sample across said porous substrate may be comprised between 10 min and 1 sec. Usually, duration of one cycle is comprised between 5 min and 10 sec. More usually, duration of one cycle is comprised between 5 min and 10 sec. Yet more usually, duration of one cycle is comprised between 1 min and 45 sec. A particular suitable example of duration of a single cycle of forward and backward flow is 30 sec.

An optimum flow rate is such that the residence time of the analyte molecules near the immobilized target-molecules is sufficient to generate interaction events of measurable quantities of labeled analyte molecules in the shortest possible time.

It might be desirable, however, to manipulate the flow rate to provide for the monitoring of interaction events between target-molecules and analytes with pre-defined kinetic properties; e.g. high $k_{ass}$ values. For example, with high flow rates, only those analyte molecules with very fast on-rates and slow off-rates or which rebind quickly form target-molecule/analyte complexes on the substrate. Flow rate may be increased or decreased with a time factor two; i.e. e.g. from 1 cycle/15 sec to 1 cycle/30 sec to 1 cycle/60 sec and so on. Equally suitable are time factor increments of 4, 6, 8, or 10.

In one embodiment of the present invention, a method as described herein is provided, wherein said measuring and monitoring is in function of flow speed.

It is common to perform analysis at a single constant temperature; the preferred temperature will depend on the application. Typically, thermophilic cell-based applications are performed at a temperature chosen from 0˚C to 100˚C, typical physiological assays are performed at 37˚C, room-temperature assays require temperatures from 20˚C to 30˚C.

Adjustment of reaction temperature may be accomplished by coupling of the substrate via a substrate holder to a heating device such as e.g. a waterbath or a conductive heating plate.

However, it might be desirable, to vary temperature as incubation of analyte molecules with target-molecules proceed in time. Such temperature variation experiment allows specificity analysis of the interacting molecules. The temperature of the substrate may be stepwise raised by any convenient increment. A suitable temperature increment is ranged between 20°C and 1°C. More suitable temperature increments are ranged between 15°C and 2°C; 10°C and 5°C; including the outer limits. Additionally, temperature variation may be continues.

Accordingly, in one embodiment of the present invention, a method as described herein is provided, wherein measuring and monitoring is in function of temperature.

Accordingly, in another embodiment of the present invention, a method as described herein is provided, wherein a reaction parameter is chosen from the group comprising temperature, flow speed, pH, and ionic strength.

In a further embodiment of the present invention, a method as described herein is provided, wherein varying of a reaction parameter is gradual.

In addition, the method of the present invention allows for continues as well discontinues monitoring of interactions between target-molecules and analyte.

Accordingly, in one embodiment of the present invention, a method as described herein is provided, wherein measuring and monitoring is in real time.

In another embodiment of the present invention, a method as described herein is provided, wherein measuring and monitoring is in semi-real time.

In the present invention, a first binding substance or a target-molecule is immobilized on the substrate at a spatially predefined region, i.e. at a particular spot. The term "first binding substance" as used in this specification refers to any molecule directly bound to the substrate such as activating groups including linkers, and target-molecules.

In one embodiment a method as described herein is provided, wherein said porous substrate comprises a plurality of target-molecules within predefined regions on said porous substrate.

In a further embodiment a method as described herein is provided, wherein said predefined regions are spatially arranged to form microarrays.

The microarrays of the present invention may be of any desired size, from two spots to $10^6$ spots or even more. The upper and lower limits of the size of the substrate are determined solely by the practical considerations of working with extremely small or large substrates.

The terms "predefined region" or "spot" are used interchangeably throughout the present invention. The latter terms relate to individually, spatially addressable positions on an array.

A predefined region is a localized area on a substrate which is, was, or is intended to be used for the attachment, directly or by synthesis, a target-molecule. The predefined region may have any convenient shape, e.g., circular, rectangular, elliptical, wedge-shaped, etc. A predefined region and, therefore, the area upon which each distinct target-molecule is attached, is smaller than about 1 cm$^2$ or less than 1 mm$^2$. Usually, the regions have an area of less than 10.000 $\mu$m$^2$, or more usually less than 100 $\mu$m$^2$ and may be less than 10$\mu$m$^2$. Within these predefined regions, the target-molecule therein attached is in a substantially pure form.

With "substantially pure" is meant exhibiting characteristics that distinguishes it from other predefined regions. Typically, purity may be measured in terms of biological activity or function as a result of uniform sequence. Such characteristics will typically be measured by way of binding with a selected receptor. Usually, the region is sufficiently pure such that the predominant species in the predefined region is the desired species.

The expression "spatially arranged" to form distinct arrays means that the predefined regions on the substrate, onto which the target-molecules attach, are laid out in precise patterns, such as rows of dots, or rows of squares, or lines.

The term "microarray" as used in the present specification refers to a porous substrate, with a matrix of target-molecules arrayed at specific positions.

The expressions "attached to" or "adhered to" or "bound to" a porous substrate denote that said target-molecules are directly or indirectly fixed or immobilized to the substrate and that more than 95% of the target-molecules remain associated with the substrate at least until all the manipulations are completed and the level of binding is assessed.

The expression "immobilized on a porous substrate" as used in the present specification refers to the attachment or adherence of one or more target-molecules to the surface of a porous substrate including attachment or adherence to the inner surface of said substrate.

Molecules or compounds may be immobilized either covalently (e.g., utilizing single reactive thiol groups of cysteine residues,) or non-covalently but specifically (e.g., via immobilized antibodies, the biotin/streptavidin system, and the like), by any method known in the art. Further examples of the various methods that are available to attach target-molecules to porous substrates include but are not limited to biotin-ligand non-covalently complexed with streptavidin, S-H-ligand covalently linked via an alkylating reagent such as an iodoacetamide or maleimide which reacts with SH and forms a thioether bond, amine-ligand covalently linked via an activated carboxylate group (e.g., EDAC coupled, etc.), phenylboronic acid (PBA)-ligand complexed with salicylhydroxamic acid (SHA), and acrylic linkages allowing polymerization with free acrylic acid monomers to form polyacrylamide or reaction with SH or silane surfaces. More specifically, immobilization of proteins may be accomplished through attachment agents selected from the group comprising cyanogen bromide, succinimides, aldehydes, tosyl chloride, avidin-biotin, photo-crosslinkable agents including hetero bi-functional cross-linking agents such as N-[y-maleimidobutyryloxylsuccinimide ester (GMBS), epoxides, and maleimides. Antibodies may be attached to a porous substrate by chemically cross-linking a free amino group on the antibody to reactive side groups present within the support. For example, antibodies may be chemically cross-linked to a substrate that contains free amino, carboxyl, or sulfur groups using glutaraldehyde, carbo-di-imides, or hetero bi-functional agents such as GIVMS as cross-linkers.

The methods as described herein for monitoring interactions between target-molecules and analytes allow the determination of interaction parameters including binding constants as well as dissociation constants.

Accordingly, in one embodiment of the present invention, an excess of unlabeled analyte molecules is added.

In a further embodiment, said excess of unlabeled analyte molecules is added after completion of immobilized target-molecule/analyte complex formation.

The addition of unlabeled analyte molecules after target-molecule/labeled analyte complex formation result in dissociation of the labeled analyte, which will be replaced by the unlabeled analyte-molecule, which prevents so-called rebinding. Rebinding often leads to an underestimation of the dissociation constant.

A further aspect of the present invention relates to use of a method as described herein to study analyte-molecule binding kinetics.

Another aspect of the present invention relates to se of a method as described herein for measuring dissociation constants of ligand-receptor pairs.

Yet another aspect of the present invention relates to use of a method as described herein for screening therapeutic compound libraries.

Yet another aspect of the present invention relates to use of a method as described herein for screening diagnostic protein libraries.

Another aspect of the present invention relates to the use of a porous substrate for the preparation of a microarray kit for carrying out a method according to the present invention.

## Short Description of the Figures

Figure 1 illustrates a lay-out of a Llama VHH antibody array as used in Example 1. The array comprises 7 VHH proteins designated C1, C4, E7, C12, B5, H2 and G11 in double; a number of PBS negative controls and a Fluorescein-labeled reference oligonucleotide.

Figure 2 illustrates the detection results of Cy3-labeled total IgG contacted with an antibody array as shown in Figure 1 of Llama VHH antibody fragments as described in Example 1. Total IgG and IgG1 are used at concentrations of 1 μg/ml (6.7 nM). Exposure times range from 55 ms (Figure 2A) to 110 ms (Figures 2C and 2D) to 220 ms (Figure 2B).

- Figure 2A illustrates the results obtained with total IgG after one cycle of flow-through pumping;
- Figure 2B illustrates the results obtained with IgG1 after 2 cycles of flow-through pumping;
- Figure 2C illustrates the results obtained with total IgG after 120 cycles of flow-through pumping (1 hour duration); and
- Figure 2D illustrates the results obtained with IgG1 after 120 cycles of flow-through pumping (1 hour duration).

Figure 3 illustrates the concentration dependent binding of total IgG to VHH E7 (see Example 1). au, arbitrary unit.

Figure 4 illustrates the kinetics of binding of total IgG (1 nM) to immobilised VHH's C4 and E7 (see Example 1).

Figure 5 illustrates the influence on background fluorescence of the addition of serum to a Mab57.9 antibody-complex prior to contacting with a peptide array as described in Example 2:

- Figure 5A illustrates the fluorescence result in absence of serum;
- Figure 5B illustrates the fluorescence result after addition of 10% serum;
- Figure 5C illustrates the fluorescence result after addition of 20% serum; and
- Figure 5D illustrates the fluorescence result after addition of 40% serum.

Camera set-up: gain 255, gamma 100, exposure time 770 ms.

**Figure 6** illustrates the influence on the background fluorescence of the addition of serum to a Mab3418 antibody-complex prior to contacting to a peptide array as described in Example 2:

- Figure 6A illustrates the fluorescence result in absence of serum;
- Figure 6B illustrates the fluorescence result after addition of 10% serum;
- Figure 6C illustrates the fluorescence result after addition of 20% serum; and
- Figure 6D illustrates the fluorescence result after addition of 40% serum.

Camera set-up: gain 255, gamma 100, exposure time 77.8 ms.

**Figure 7** illustrates the influence of addition of serum on complex formation of Mab57.9 and goat-anti-mouse Fluorescein. Exposure time is 440 ms.

**Figure 8** illustrates the detection of interaction between Mab3418/gam-Flu antibody and 6 different peptides in the absence of serum.

**Figure 9** illustrates the detection of interaction between Mab3418/gam-Flu antibody and 6 different peptides in the presence of 10% serum.

**Figure 10** illustrates an array lay-out as used in Examples 3 and 4 (in double: rows A to D and E to H are the same). The array comprises PBS negative controls and a reference oligo.

**Figure 11** illustrates the fluorescence results after incubation of various Fluorescein-labeled antibodies with an IgG array as shown in Figure 10 and as described in Example 3. The exposure time is 220 ms.

- Figure 11A illustrates the binding of anti-mouse IgG to mouse IgG and rat-IgG,
- Figure 11B illustrates the binding of anti-goat IgG to goat IgG and bovine IgG, and
- Figure 11C illustrates the binding of anti-rat IgG to all spotted IgG's.

**Figure 12** illustrates the fluorescence results after incubation of various Cy3-labeled antibodies with an IgG array as shown in Figure 10 and as described in Example 3. The exposure time is 55 ms.

- Figure 12A illustrates the binding of anti-mouse IgG to mouse IgG and rat-IgG,

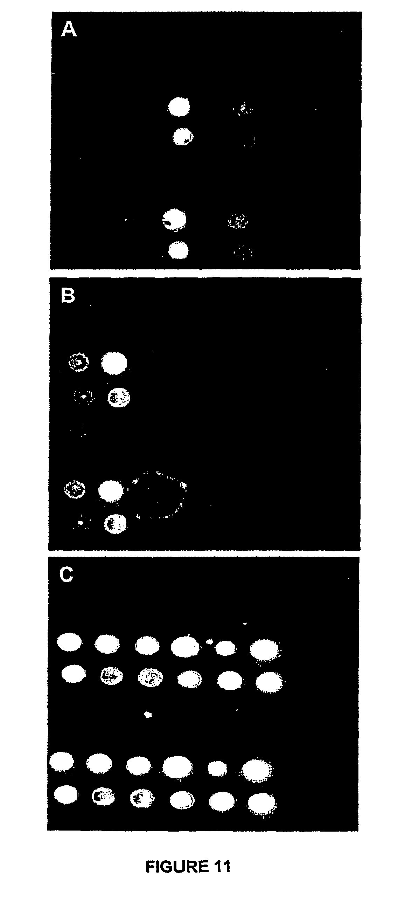

- Figure 11B illustrates the binding of anti-goat IgG to goat IgG and bovine IgG, and
- Figure 11C illustrates the binding of anti-rat IgG to mouse IgG and rat IgG.

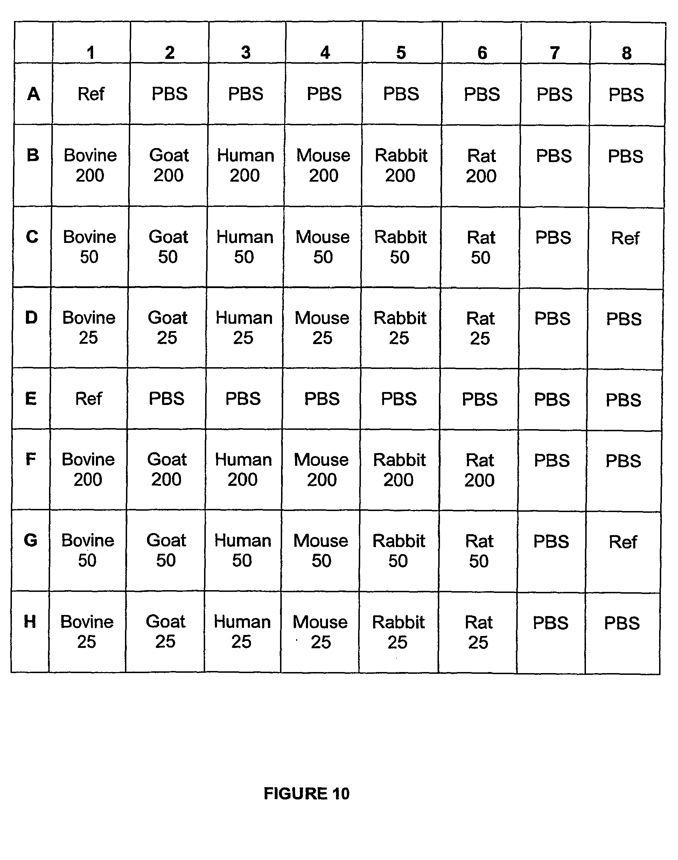

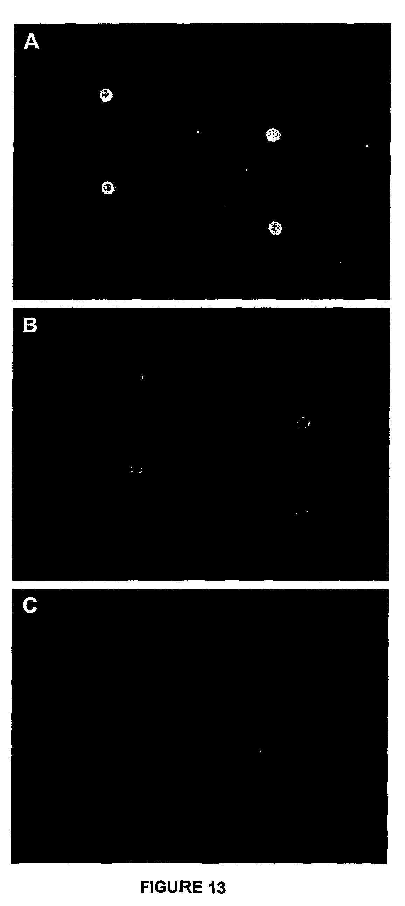

**Figure 13** illustrates the results obtained after incubation of an IgG array as shown in Figure 10 with Fluorescein-labeled anti-goat IgG. The concentration of Fluorescein-labeled anti-goat IgG is 0.02 μg/ml. Exposure time is 220 ms.

- Figure 13A illustrates detection immediately after washing of the array;
- Figure 13B illustrates detection at 5 min after washing; and
- Figure 13C illustrates detection at 50 min after washing.

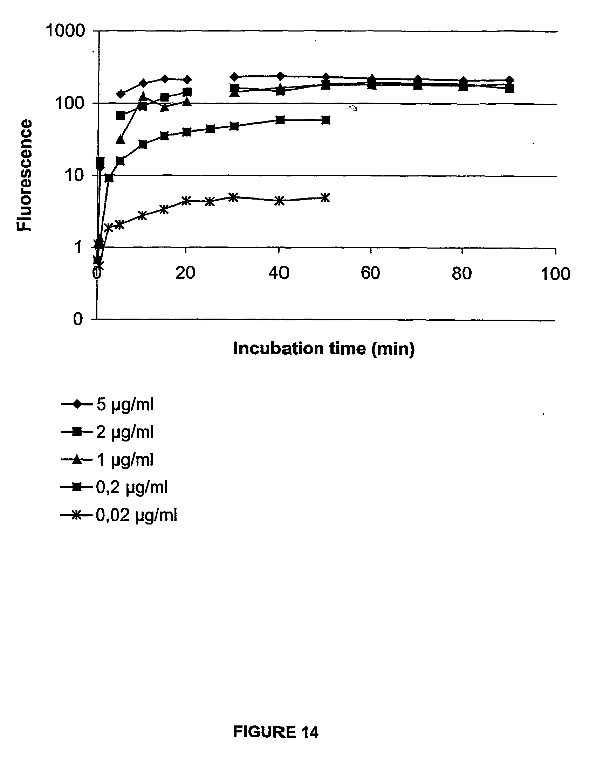

**Figure 14** illustrates the binding of Fluorescein-labeled anti-goat IgG in different concentrations to goat IgG as described in Example 4.

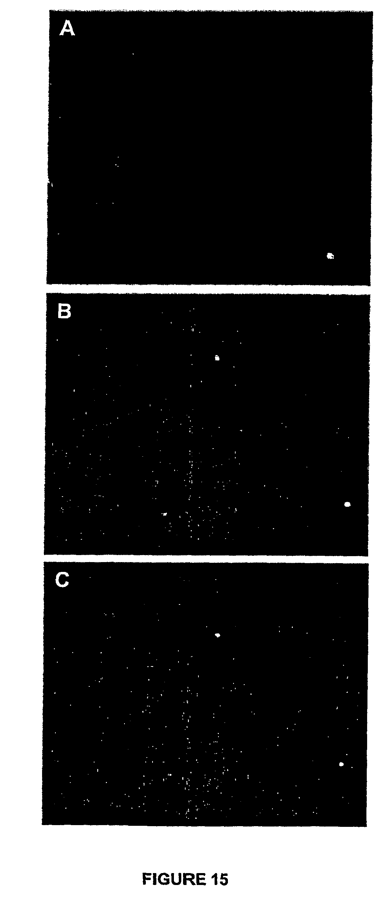

**Figure 15** illustrates the binding of Cy3-labeled anti-goat IgG at low concentration to an array of various IgG's (see Figure 10) as decribed in Example 4. The concentration of Cy3-labeled anti-goat is 0.002 μg/ml. Exposure time is 990 ms.

- Figure 15A illustrates detection after immediately after washing of the array;
- Figure 15B illustrates detection at 60 min after washing; and
- Figure 15C illustrates detection at 150 min after washing.

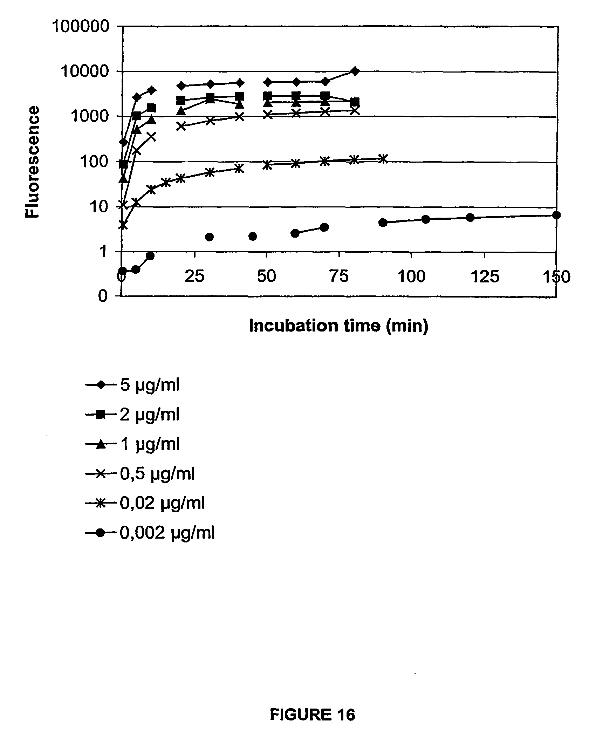

**Figure 16** illustrates the binding of Cy3-labeled anti-goat IgG at different concentrations to goat IgG as described in Example 4.

**EXAMPLES**

The following examples of the invention are exemplary and should not be taken as in any way limiting.

**Example 1: Rapid and specific detection of protein-protein interactions using an antibody array of Llama VHH antibody fragments immobilised on a solid substrate**

Llama single domain antibody fragments (VHH's) were used in the detection of proteins. Immunoglobulins of the G-type are the proteins of interest and were used in the selection of the VHH's. VHH's are very applicable in the generation of microarrays for a number of reasons; among others high stability, easy molecular cloning (small, approximately 125 amino acids) and high solubility.

*1.1. Isolation of VHH proteins*

Phage display technology was used to isolate the VHH proteins. The VHH proteins were selected from libraries that were generated from RNA of two Llama's immunised with either IgG1 and IgG2a or a mixture of Fc fragments from human and mouse immunoglobulins. Successive rounds of selection yielded 7 VHH proteins with specificity or cross-reactivity towards mouse IgG subclasses.

*1.2. Preparation of the microarray*

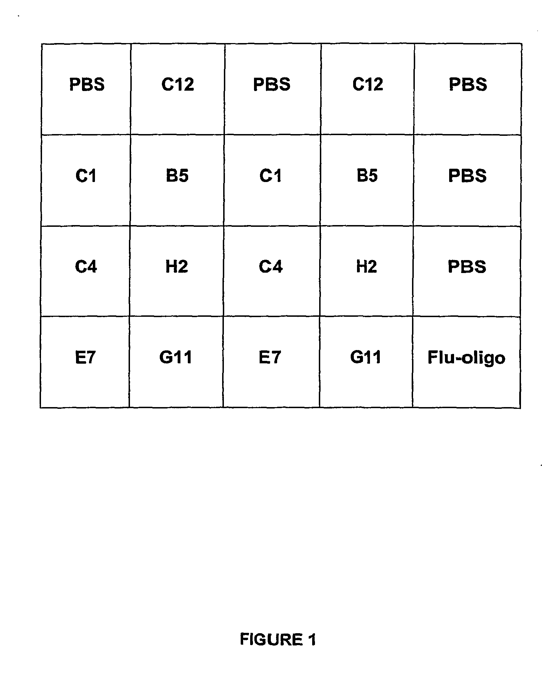

The VHH proteins, comprising a C-terminal cysteine residue (enabling covalent coupling to maleimide groups), were spotted in an array format (Figure 1) at a 10 $\mu$M concentration on a maleimide-modified solid support (ANOPORE™ aluminum-oxide membrane; Cat. No. 68090084, Whatman Scientific Ltd.).

*1.3. Contacting the VHH array with Cy3-labeled mouse total IgG or IgG1 and detection of signal*

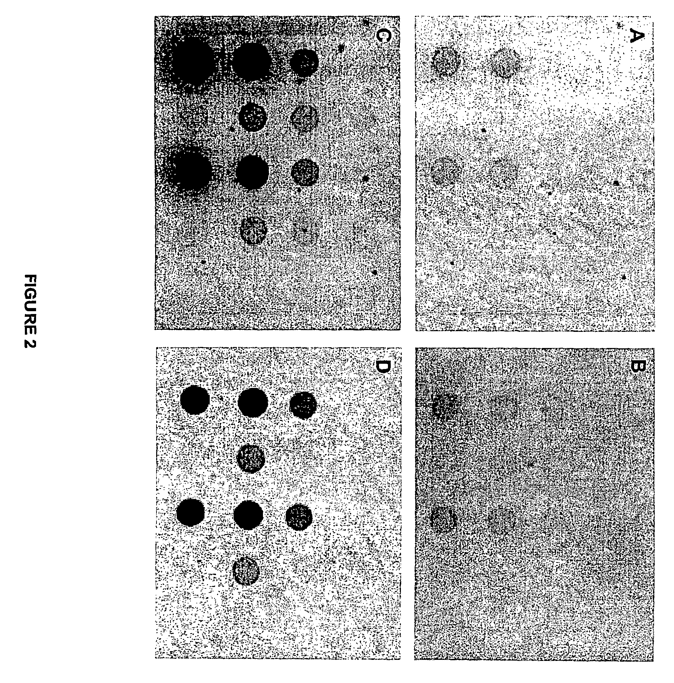

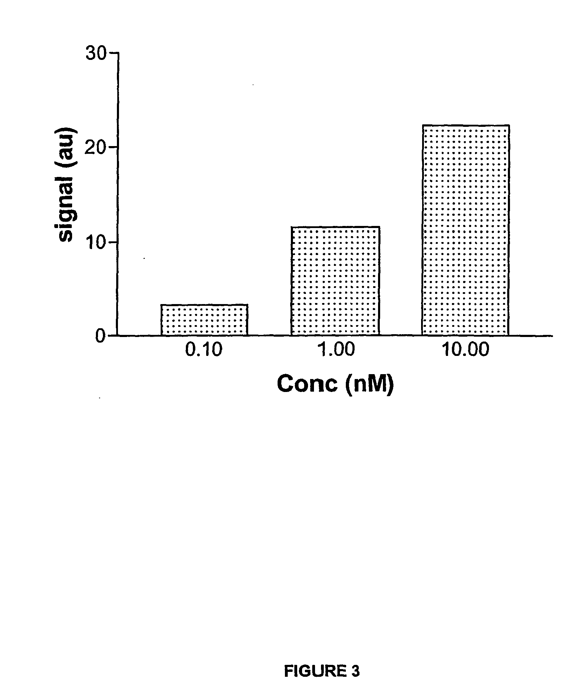

After extensive washing, the membrane was incubated with Cy3-labeled mouse total IgG (total immunoglobulin G) or IgG1 (subclass) at a 1 $\mu$g/ml concentration (6.7 nM) in phosphate buffered saline (PBS) supplemented with Tween-20 (0.05%).
In both cases signal was readily detected at particular VHH-spots (e.g. C4 and E7) after 1 or 2 times of flow-through pumping of the sample (Figures 2a and 2b). After 1 hour of incubation, while flow-through pumping according to a fast-incubation procedure (1 cycle/0.5 min; sample passes immobilised VHH 4 times per minute), strong signals were recorded (see Figure 2c and 2d). Signals from distinct VHH-spots varied to a large extent, indicating specific recognition of the immunoglobulin by the immobilised VHH proteins. Binding is concentration dependent and is shown for the binding of total IgG to VHH in Figure 3 (see E7). The lowest level of total IgG detected was 0.1 nM in a 20 $\mu$L sample (2 femtomole).

*1.4. Binding kinetics*

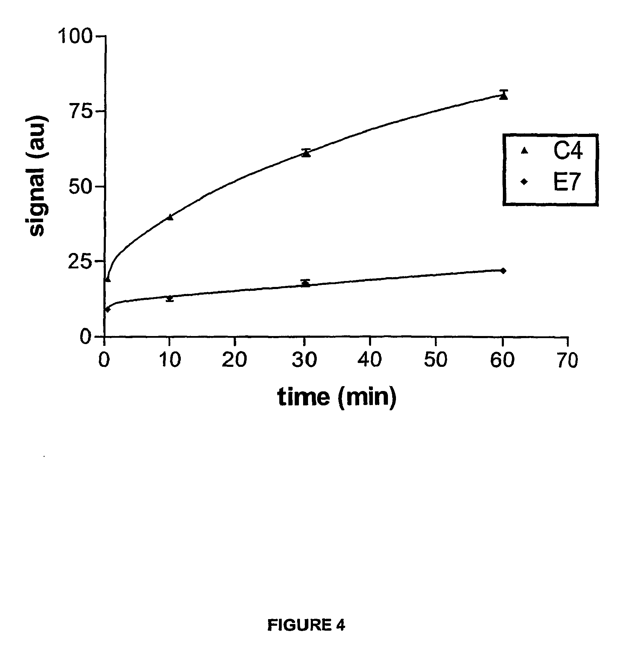

The detection of the signals from binding Cy3-labeled total IgG as obtained in Example 1 was recorded in time to study the binding kinetics. Figure 4 shows the association curves of the binding of Cy3-labeled total IgG (1 nM) to VHH C4 and VHH E7, respectively. By fitting the data and using Formula 1, the rate constants ($k_{ass}$) were determined (see Table 1).

$$B = B_{max} \cdot [1-exp(-k.t)]$$

**Formula 1**

Even for low concentrations of VHH proteins, e.g. for a VHH protein (B5) spotted from a solution of 3 $\mu$M whereby less than 0.9 femtomole VHH was immobilised, a signal was obtained after incubation with total IgG.
The above-mentioned results demonstrate that the present invention allows for rapid and specific detection of interactions between immunoglobulins and Llama VHH proteins immobilised on a solid support. The methods according to the present invention enables kinetic studies of molecule interactions as well.

**Example 2: Detection of antibody-peptide interactions in the presence of serum**

The influence of addition of serum before or after complex formation between antibody and Fluorescein-labeled second antibody was analysed.

### 2.1. Strategy

Fresh peptide-arrays were prepared using standard methods; the different peptides that were used are presented in Table 2. Two antibodies (Mab3418, 31 mg/ml and Mab57.9, 1.87 mg/ml; Pepscan Systems) were pre-incubated with a Fluorescein-labeled goat anti-mouse antibody. Serum (ABV-HIV-MCV-; obtained from P. Schneeberger, BMC) was added before (Mab3418: 0% and 10% serum, Mab57.9: 0%, 10%, 20% and 40% serum) or after (0%, 10%, 20% and 40% serum) antibody-complex formation. After pre-incubation the antibody-complex was diluted in PBS/Tween (25x; Organon) to a final concentration of 5 $\mu$g/ml and 0.3 $\mu$g/ml respectively. The diluted antibody-complex was added to the peptide array and incubated dynamically at 37˚C. The Mab3418-complex was incubated during 15 minutes and the Mab57.9-complex was incubated during 25 minutes. Images were made at different time points (camera set up with MAS device; Hamilton pump and water bath; Olympus microscope Bx41, Tokyo Japan, CCD camera Kappa type PS2, serial no. DXP1206, Fabr. no. 0280).

### 2.2. Influence of serum on binding of the antibodies to the peptide array

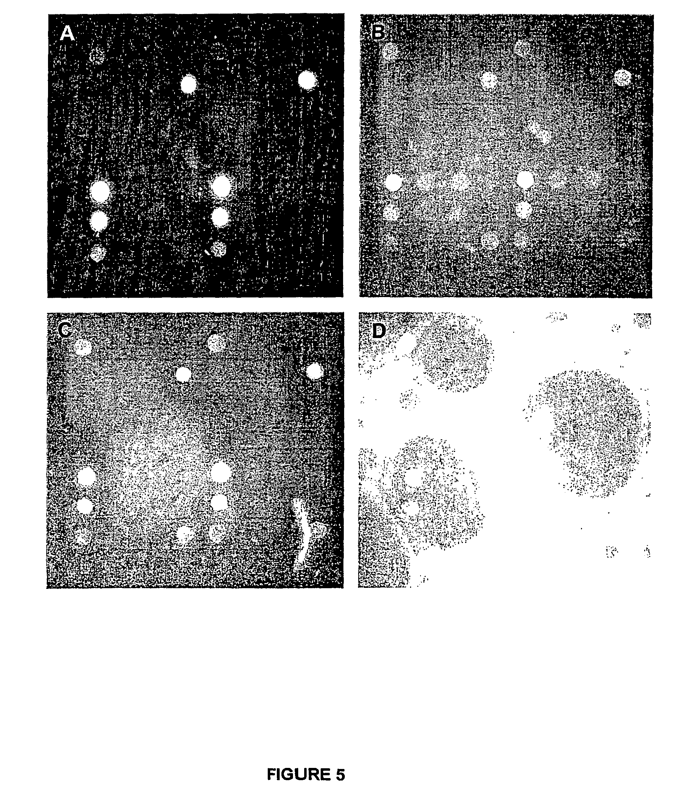

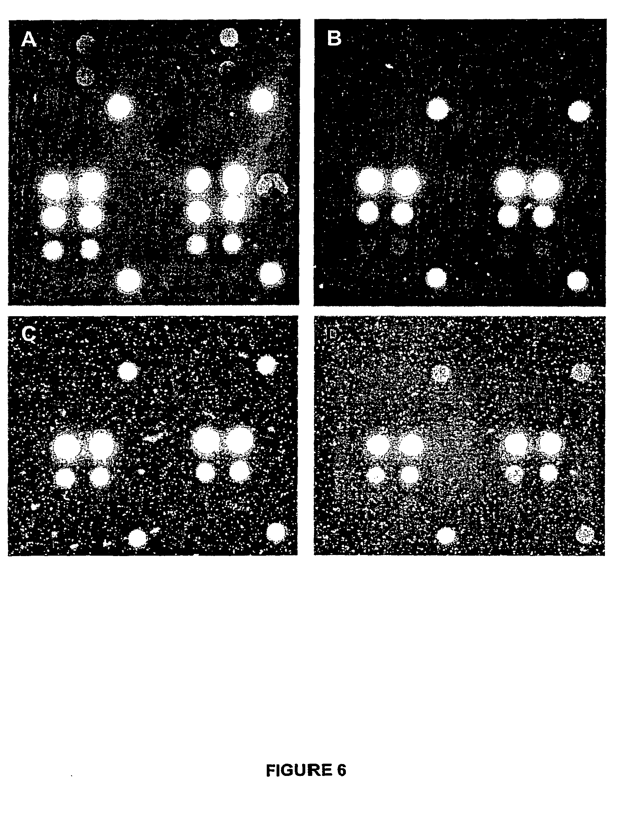

Images were made at 15 minutes after incubation and are shown in Figures 5 and 6. The background fluorescence is increasing by the addition of serum to the antibody-complex. More serum resulted in more background. Also the fluorescence of the spots decreased with increase of serum.

### 2.3. Influence of *serum on complex formation*

### Mab57.9 antibody-Fluorescein complex

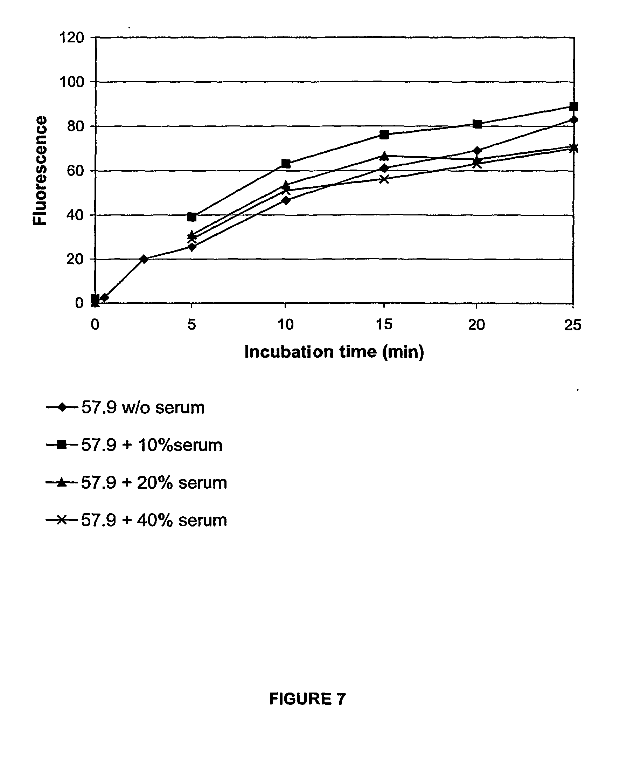

The addition of serum did not have a big influence on the formation of the Mab57.9 antibody-Fluorescein complex and the subsequent binding of the complex to the peptide array. Figure 7 displays comparative results. Table 3 represents a fluorescence analysis on a 180 $\mu$M MDG21 peptide spot.

### Mab3418 antibody-Fluorescein complex

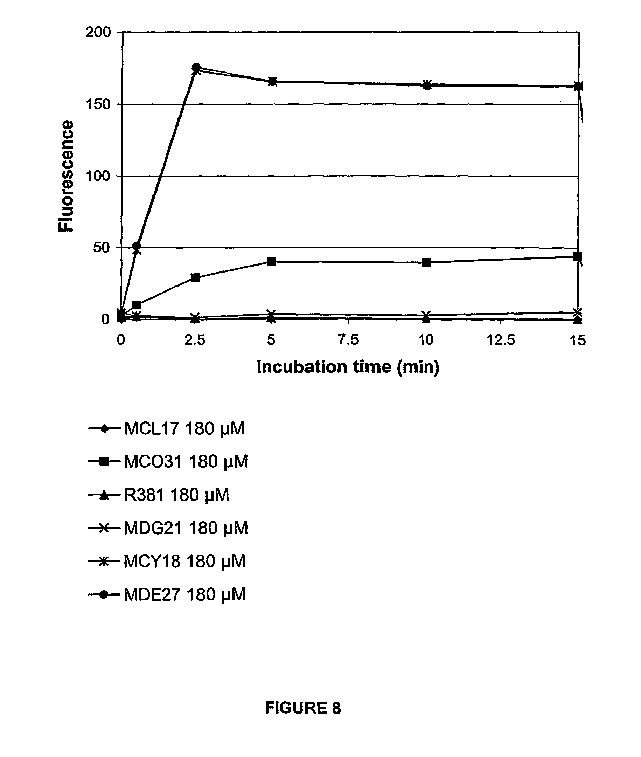

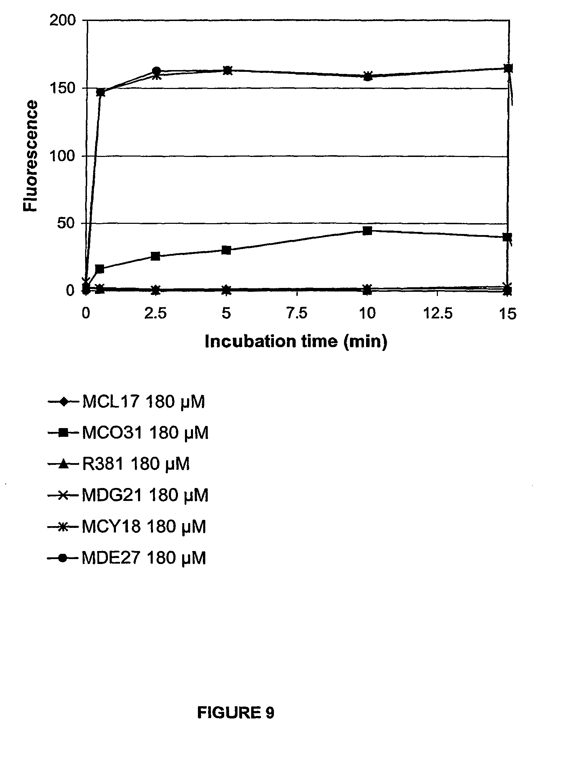

The addition of 10% serum did not have an influence on the formation of the Mab3418 antibody-Fluorescein labeled secondary antibody complex and the subsequent binding of the complex to the peptide array. The results are shown in Figures 8 and 9.

The above-mentioned results demonstrate that the addition of serum after complex formation between antibody and labeled second antibody resulted in decreased signals upon incubation of the complex with the peptide array. Addition of serum during complex formation between antibody and labeled second antibody did not influence the strength of the signals when the complex was incubated with the peptide array. Background fluorescence was higher when serum was added to the labeled antibody complex.

### Example 3: Analysis of absorptive coupled antibodies on an Anopore aluminum oxide membrane.

The detection of different kind of absorptive-coupled IgG's on an Anopore membrane (0.2 $\mu$m, Cat. No. 68090084, Whatman) upon incubation with anti-IgG antibodies labeled with Fluorescein or Cy3 was tested.

### 3.1. Preparation of IgG arrays and antibody labeling

IgG-arrays were prepared by spotting (Biochip Arrayer; Packard Biochip Technologies) 200 $\mu$M, 50 $\mu$M and 25 $\mu$M solutions of IgG's (Sigma; see Table 4) on a solid support. The array layout is shown in Figure 10. Anti-IgG antibodies (rat, goat and mouse anti-IgG, Sigma; see Table 4) were labeled with Fluorescein-succinimmidyl-ester (5-FAM SE; Cat. No. C2210, Molecular Probes) or with Cy3 using the Fluorolink-Ab-Cy3 labeling kit (Amersham, Arlington Heights, IL) according to the manufacturer's guidelines. After labeling the antibodies were purified using Sephadex G25 columns. The concentration of the labeled antibodies was determined by using the Micro BCA™ Assay (Pierce). To determine the degree of labeling the labeled antibodies were measured with a spectrophotometer at wavelengths: 280 nm (protein), 494 nm (Fluorescein) and 552 nm (Cy3). Table 5 shows the result of the degree of labeling.

### 3.2. Incubation with Fluorescein-labeled antibodies

The labeled antibodies were diluted in PBS/Tween to a final concentration of 1.8 μg/ml. The diluted antibody-complex was added to the IgG-array and incubated dynamically at 37°C during 40 minutes. Images were taken at several time points during the incubation.

Figure 11 shows the binding of Fluorescein-labeled antibodies to the IgG's on the array after 40 minutes of incubation. The anti-goat and anti-mouse antibodies gave a result that could be expected: anti-mouse IgG-Fluo binds with mouse-IgG and rat-IgG, anti-goat IgG-Fluo binds with goat-IgG and bovine-IgG. A test for cross-reactivity of anti-mouse IgG with human IgG showed a negative result. Cross-reactivity with rat-IgG was not unlikely. In this experiment the anti-rat antibody unexpectedly bonded with all the spotted IgG's and might have been due to the high degree of labeling with Fluorescein.

### 3.3. Incubation with Cy3-labeled antibodies

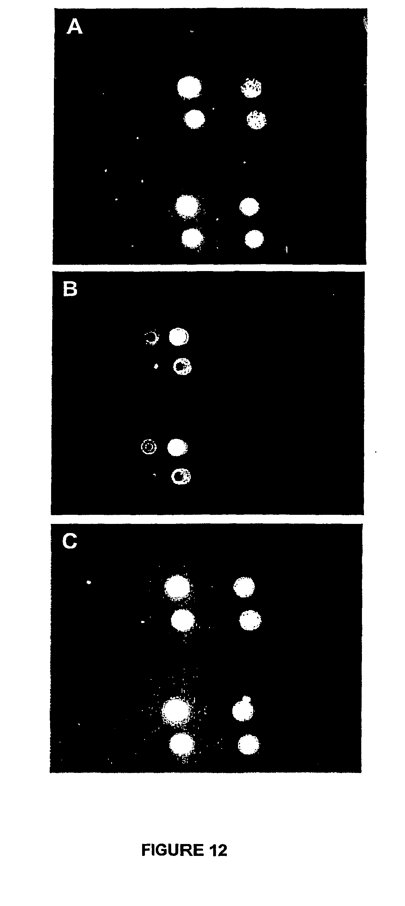

Figure 12 shows the binding of Cy3-labeled antibodies to the IgG's on the array after 40 minutes of incubation. The anti-mouse and anti-goat antibodies labeled with Cy3 gave about the same results as the same antibodies labeled with Fluorescein. The anti-rat antibody labeled with Cy3 gave much more specific results, although the antibody still bonded stronger to mouse IgG than to rat IgG. The degree of labeling with Cy3 was half the amount of the degree of labeling with Fluorescein. Therefore the degree of labeling may determine the specificity of the antibody.

Comparison of the signals of the above-described experiments with Fluorescein- and Cy3-labeled antibodies show that the signals of Cy3 are a factor 10 stronger than the signals of Fluorescein.

It has been shown that it is possible to detect absorptive-coupled IgG's on a solid support with Cy3- or Fluorescein-labeled anti-IgG's. The binding of the labeled antibodies was most specific when the degree of labeling was below 6.

### Example 4: Determination of the detection limit of a multiple-antibody array

The detection limit of antibody-antibody interactions was determined on an Anopore aluminum-oxide support (Cat. No. 68090084, Whatman Scientific Ltd.) and the sensitivities of Cy3-and Fluorescein-labeled antibodies were compared.

### 4.1. Preparation of IgG arrays and antibody labeling

The IgG-arrays of Example 3 were used for the following experiments. The array layout is shown in Figure 10. Also the Cy3- and Fluorescein-labeled anti-goat IgG's of Example 3 were used for these experiments

The labeled antibodies were diluted in PBS/Tween to final concentrations of 0.02 μg/ml, 0.2 μg/ml, 1 μg/ml, 2 μg/ml, and 5 μg/ml for Fluorescein-labeled anti-goat IgG and 0.002 μg/ml, 0.02 μg/ml, 0.5 μg/ml, 1 μg/ml, 2 μg/ml, and 5 μg/ml for Cy3-labeled anti-goat IgG. The diluted antibody-complex was added to the IgG-array and incubated dynamically at 37°C during maximal 150 minutes. Images were made at several time points during incubation.

### 4.2. Incubation with Fluorescein-labeled anti-goat IgG:

Figure 13 shows the results of the lowest concentration of Fluorescein-labeled anti-goat IgG tested on the IgG array. 0.02 μg/ml of Fluorescein-labeled anti-goat IgG was observed to be the lowest concentration that gives a binding-signal on the array within a reasonable time (i.e. after 5 minutes the signal can be seen), with a reasonable exposure time. The results of binding of the different concentrations of Fluorescein-labeled anti-goat IgG to goat IgG (50 μM) are shown in Figure 14.

### 4.3. Incubation with Cy3 labeled anti-goat IgG:

Figures 15 and 16 show the results of the lowest concentration of Cy3-labeled anti-goat igG tested on the array. Longer exposure times (e.g. 2.4 s) showed already signals at 30 minutes incubation but gave a very high background fluorescence. 0.002 μg/ml Cy3-labeled anti-goat IgG was observed to be the lowest concentration that gave a binding-signal on the array within a reasonable time.

The results of binding of different concentrations of Cy3-labeled anti-goat IgG to goat IgG (50 μM) are shown in Figure 16.

The above-described experiments show that the detection limit for Fluorescein-labeled anti-goat IgG is about 0.02 μg/ml (0.13 nM) and the detection limit for Cy3-labeled anti-goat IgG is about 0.002 μg/ml (13 pM).

**Table 1.**

|  |  | VHH C4 | VHH E7 |
|---|---|---|---|
| Variables | $B_{max}$ k | 67.57 0.1079 | 17.52 0.3956 |
| Standard Error | $B_{max}$ K | 0.1675 0.001192 | 0.03615 0.007711 |
| Goodness of fit | $R^2$ | 0.9822 | 0.9113 |

**Table 2.**

| Peptide | AA sequence | Purity (%) | kD | Mw (g/mol) |
|---|---|---|---|---|
| MCL17 | GCASLQGMDTCGK | 60 | $10^{-3}$ | 1331 |
| MCY18 | APDPFKQGVDTCGK | 90 | $4^{-6}$ | 1503 |
| MCO31 | CAFKQGVDTCGK | 30 | $3^{-7}$ | 1298 |
| MDE27 | GCAPDPFKQGVDTCGK | 80 | $2^{-8}$ | 1663 |
| R381 | Pepscan Systems | 60 |  | 1910 |
| MDG21 | Pepscan Systems | 90 |  | 1868 |

Table 3.

| Incubation time (min) | Fluorescence | | | |
|---|---|---|---|---|
|  | 57.9 w/o serum | 57.9 + 10% serum | 57.9 + 20% serum | 57.9 + 40% serum |
| 0 | 1 | 2 | 0 | 0 |
| 0.5 | 3 |  |  |  |
| 2.5 | 20 |  |  |  |
| 5 | 26 | 39 | 31 | 29 |
| 10 | 47 | 63 | 54 | 51 |
| 15 | 61 | 76 | 67 | 56 |
| 20 | 69 | 81 | 65 | 63 |
| 25 | 83 | 89 | 71 | 70 |

**Table 4**

| IgG | Company | Catalogue No |
|---|---|---|
| Bovine | Sigma | 15506 |
| Goat |  | 15526 |
| Human |  | 14506 |
| Mouse |  | 15381 |
| Rabbit |  | 15006 |
| Rat |  | 14161 |

(continued)

| Anti-IgG | | |
|---|---|---|
| Anti-Bovine | Sigma | B6901 |
| Anti-Goat | | G9019 |
| Anti-Human | | 16135 |
| Anti-Mouse | | M6898 |
| Anti-Rabbit | | R2004 |
| Anti-Rat | | R5310 |

**Table 5.**

| Labeled antibody | Degree of labeling |
|---|---|
| Anti-Rat IgG-Fluo | 11 |
| Anti-Goat IgG-Fluo | 2 |
| Anti-Mouse IgG-Fluo | 1 |
| Anti-Rat IgG-Cy3 | 6 |
| Anti-Goat IgG-Cy3 | 2 |
| Anti-Mouse IgG-Cy3 | 7 |

**Claims**

1.  A method for monitoring interactions between target-molecules and at least one analyte comprising:

    (a) contacting a plurality of target-molecules, said target-molecules being immobilized on a porous substrate with a sample, said sample comprising at least one analyte;
    (b) incubating said target-molecules with said sample under conditions supporting target-molecule/analyte interaction ;
    (c) monitoring said interaction as defined in step (b), said monitoring being in function of time of incubation as defined in step (b);
    (d) optionally determining at least one interaction parameter;
    (e) varying said conditions as defined in step (b) by varying a reaction parameter, wherein said reaction parameter is chosen from the group comprising temperature, flow speed, pH and ionic strength, and;

(f) repeating, at least once, steps (c) to (e).

2. A method according to claim 1, wherein said porous substrate is a flow-through substrate.

3. A method according to any of claims 1 - 2, wherein said porous substrate is a metal oxide substrate.

4. A method according to any of claims 1-3, wherein said porous substrate is an aluminum oxide substrate.

5. A method according to any of claims 1-4, wherein said incubating is dynamic and wherein said dynamic incubation comprises subjecting said porous substrate to at least one cycle of forward and backward flow of sample across said porous substrate, wherein said forward and backward flow is established by application of alternating positive and negative pressure.

6. A method according to any of claims 1 - 5, wherein said interaction parameter is chosen from the group comprising specificity, affinity, association constant, absorption parameter, distribution parameter, metabolism parameter and excretion parameter.

7. A method according to any of claims 1 - 6, wherein said monitoring comprises the steps of:

   (a) detecting a signal, said signal resulting from an interaction as defined in step (b) of claim 1, and;
   (b) recording the level of said signal as defined in step (a), said level being indicative for the strength of target-molecule/analyte interaction.

8. A method according to any of claims 1 - 7, wherein said measuring and monitoring is in real time.

9. A method according to any of claims 1 - 8, wherein said measuring and monitoring is in semi-real time.

10. A method according to any of claims 1 - 9, wherein said measuring and monitoring is in function of temperature.

11. A method according to any of claims 1 - 10, wherein said measuring and monitoring is in function of flow speed.

12. A method according to any of claims 1 - 11, wherein said varying of said reaction parameter is gradual.

13. A method according to any of claims 1 - 12, wherein said target-molecules are selected from the group comprising hormone receptors, peptides, enzymes, oligonucleotides, nucleic acids, oligosaccharides, proteins, monoclonal antibodies, antibody fragments, haptens, and aptamers.

14. A method according to any of claims 1 - 13, wherein said analyte is selected from the group comprising antibodies, monoclonal antibodies, antibody fragments, antisera, polynucleotides, nucleic acids, peptides, enzyme binding sites, polysaccharides, cells, cellular membranes, and organelles.

15. A method according to any of previous claims 1 - 14, wherein said porous substrate comprises a plurality of target-molecules within predefined regions on said porous substrate.

16. A method according to claim 15, wherein said predefined regions are spatially arranged to form microarrays.

17. A method according to any of claims 1 - 16, wherein said detectable signal is selected from the group comprising a refractive index, a cellular activity, a light emission including fluorescence, a change in absorbance, a change in fluorescence, an absorbance, a color shift, a fluorescence resonance energy transfer, a radioactive emission, a change in pH, a change in temperature, and a change in mass.

18. A method according to any of claims 1 - 17, wherein said detectable signal is a light emitted signal.

19. A method according to claim 18, wherein said detectable signal is a fluorescent signal.

20. A method according to claim 19, wherein said fluorescent signal is emitted by a labeled analyte, said analyte being associated with a target-molecule.

21. A method according to any of claims 1 - 20, wherein said analyte is present in said sample in a concentration from $5 \times 10^{-4}$ nM to 10 nM.

22. A method according to claim 21, wherein said concentration is from $5 \times 10^{-3}$ nM to 1 nM.

23. A method according to claim 22, wherein said concentration is from $5 \times 10^{-2}$ nM to $10^{-1}$ nM.

24. A method according to any of claims 1-23, wherein an excess of unlabeled analyte is added.

25. A method according to claim 24, wherein said excess of unlabeled analyte is added after completion of immobilized target-molecule/analyte complex formation.

26. Use of a method according to any of claims 1-25, to study analyte-molecule binding kinetics.

27. Use of a method according to any of claims 1 - 25, for measuring dissociation constants of ligand-receptor pairs.

28. Use of a method according to any of claims 1 - 25, screening therapeutic compound libraries.

29. Use of a method according to any of claims 1 25, for screening diagnostic protein libraries.

30. Use of a porous substrate for the preparation of a microarray kit for carrying out a method according to any of claims 1-25.

**Patentansprüche**

1. Verfahren für das Überwachen von Interaktionen zwischen Zielmolekülen und mindestens einem Analyten, wobei das Verfahren folgende Schritte umfasst:

   (a) In-Kontakt-bringen einer Vielzahl von Zielmolekülen mit einer Probe, wobei die Zielmoleküle auf einem porösen Substrat immobilisiert sind, und wobei die Probe mindestens einen Analyten umfasst;
   (b) Inkubieren der Zielmoleküle mit der Probe unter Bedingungen, die die Zielmolekül/Analyteninteraktion unterstützen;
   (c) Überwachen der in Schritt (b) definierten Interaktion, wobei das Überwachen eine Funktion der Zeit der Inkubation ist, wie sie in Schritt (b) definiert ist;
   (d) gegebenenfalls Bestimmung mindestens eines Interaktionsparameters;
   (e) Verändern der in Schritt (b) definierten Bedingungen durch Verändern eines Reaktionsparameters, wobei der Reaktionsparameter aus der die Temperatur, Fließgeschwindigkeit, pH- und Ionenstärke umfassenden Gruppe ausgewählt wird und;
   (f) mindestens einmalige Wiederholung der Schritte (c) bis (e).

2. Ein Verfahren nach Anspruch 1, wobei das poröse Substrat ein Durchflusssubstrat ist.

3. Ein Verfahren nach einem der Ansprüche 1 bis 2, wobei das poröse Substrat ein Metalloxidsubstrat ist.

4. Ein Verfahren nach einem der Ansprüche 1 bis 3, wobei das poröse Substrat ein Aluminiumoxidsubstrat ist.

5. Ein Verfahren nach einem der Ansprüche 1 bis 4, wobei die Inkubation dynamisch ist, und wobei die dynamische Inkubation die Unterwerfung des porösen Substrats für mindestens einen Zyklus des Vorwärts- und Rückwärtsflusses der Probe durch das poröse Substrat umfasst, wobei der Vorwärts- und Rückwärtsfluss durch die Anwendung von alternierendem positiven und negativen Druck etabliert wird.

6. Ein Verfahren nach einem der Ansprüche 1 bis 5, wobei der Interaktionsparameter aus einer Gruppe ausgewählt wird, die Spezifität, Affinität, Assoziationskonstante, Absorptionsparameter, Verteilungsparameter, Metabolismusparameter und Absonderungsparameter umfasst.

7. Ein Verfahren nach einem der Ansprüche 1 bis 6, wobei das Überwachen folgende Schritte umfasst:

(a) Nachweisen eines Signals, wobei das Signal aus einer Interaktion resultiert, wie sie in Schritt (b) aus Anspruch 1 definiert ist, und;

(b) Aufnehmen des Niveaus des Signals, wie es in Schritt (a) definiert ist, wobei das Niveau die Stärke der Ziel-Molekül/Analyt-Interaktion aufzeigt.

**8.** Ein Verfahren nach einem der Ansprüche 1 bis 7, wobei das Messen und das Überwachen in Echtzeit erfolgen.

**9.** Ein Verfahren nach einem der Ansprüche 1 bis 8, wobei das Messen und das Überwachen in Halbechtzeit erfolgen.

**10.** Ein Verfahren nach einem der Ansprüche 1 bis 9, wobei das Messen und das Übwerwachen eine Funktion der Temperatur sind.

**11.** Ein Verfahren nach einem der Ansprüche 1 bis 10, wobei das Messen und das Überwachen eine Funktion der Flussgeschwindigkeit sind.

**12.** Ein Verfahren nach einem der Ansprüche 1 bis 11, wobei das Verändern eines Reaktionsparameters stufenweise erfolgt.

**13.** Ein Verfahren nach einem der Ansprüche 1 bis 12, wobei Zielmoleküle aus einer Gruppe ausgewählt sind, die Hormonrezeptoren, Peptide, Enzyme, Oligonukleotide, Nukleinsäuren, Oligosaccharide, Proteine, monoklonale Antikörper, Antikörperfragmente, Haptene und Aptamere umfasst.

**14.** Ein Verfahren nach einem der Ansprüche 1 bis 13, wobei der Analyt aus einer Gruppe ausgewählt ist, die Antikörper, monoklonale Antikörper, Antikörperfragmente, Antiseren, Polynukleotide, Nukleinsäuren, Peptide, Enzym-Bindestellen, Polysaccharide, Zellen, zelluläre Membrane und Organellen umfasst.

**15.** Ein Verfahren nach einem der vorhergehenden Ansprüche 1 bis 14, wobei das poröse Substrat eine Vielzahl von Zielmolekülen innerhalb vordefinierter Regionen auf dem porösen Substrat umfasst.

**16.** Ein Verfahren nach Anspruch 15, wobei die vordefinierten Regionen räumlich so angeordnet sind, dass sie Mikroarrays bilden.

**17.** Ein Verfahren nach einem der Ansprüche 1 bis 16, wobei das nachweisbare Signal aus einer Gruppe ausgewählt ist, die einen Brechungsindex, eine zelluläre Aktivität, eine Lichtemission, einschließlich Fluoreszenz, ein Wechsel in der Absorption, ein Wechsel in der Fluoreszenz, eine Absorption, eine Farbveränderung, einen Fluoreszenz-Resonanz-Energietransfer, eine radioaktive Emission, ein Wechsel des pH-Wertes, ein Wechsel der Temperatur und ein Wechsel der Masse, umfasst.

**18.** Ein Verfahren nach einem der Ansprüche 1 bis 17, wobei das nachweisbare Signal ein Licht emittiertes Signal ist.

**19.** Ein Verfahren nach Anspruch 18, wobei das nachweisbare Signal ein Fluoreszenzsignal ist.

**20.** Ein Verfahren nach Anspruch 19, wobei das Fluoreszenzsignal durch einen markierten Analyten, der mit einem Zielmolekül assoziiert ist, emittiert wird.

**21.** Ein Verfahren nach einem der Ansprüche 1 bis 20, wobei der Analyt in der Probe in einer Konzentration von $5 \times 10^{-4}$ nM bis 10 nM vorhanden ist.

**22.** Ein Verfahren nach Anspruch 21, wobei die Konzentration im Bereich von $5 \times 10^{-3}$ nM bis 1 nM vorliegt.

**23.** Ein Verfahren nach Anspruch 22, wobei die Konzentration im Bereich von $5 \times 10^{-2}$ nM bis $10^{-1}$ nM vorliegt.

**24.** Ein Verfahren nach einem der Ansprüche 1 bis 23, wobei ein Überschuss an unmarkierten Analyten zugegeben wird.

**25.** Ein Verfahren nach Anspruch 24, wobei der Überschuss an unmarkierten Analyten nach Vollendung der Bildung des immobilisierten Ziel-Molekül/Analyt-Komplexes zugegeben wird.

**26.** Verwendung eines Verfahrens nach einem der Ansprüche 1 bis 25, um die Analyt-Molekül-Bindungskinetiken zu untersuchen.

**27.** Verwendung eines Verfahrens nach einem der Ansprüche 1 bis 25, um die Dissoziationskonstanten der Liganden-Rezeptorpaare zu messen.

**28.** Verwendung eines Verfahrens nach einem der Ansprüche 1 bis 25, um therapeutische Verbindungsbibliotheken zu durchmustern.

**29.** Verwendung eines Verfahrens nach einem der Ansprüche 1 bis 25, um diagnostische Proteinbibliotheken zu durchmustern.

**30.** Verwendung eines porösen Substrates für die Zubereitung eines Mikroarray-Kits für die Durchführung eines Verfahrens nach einem der Ansprüche 1 bis 25.

## Revendications

**1.** Procédé permettant de surveiller des interactions entre des molécules cibles et au moins un analyte comprenant :

a) la mise en contact d'une multiplicité de molécules cibles, lesdites molécules cibles étant immobilisées sur un substrat poreux avec un échantillon, ledit échantillon comprenant au moins un analyte ;
b) la mise en incubation desdites molécules cibles avec ledit échantillon dans des conditions supportant une interaction molécule cible / analyte ;
c) la surveillance de ladite interaction telle que définie dans l'étape b), ladite surveillance étant fonction de la durée d'incubation telle que définie à l'étape b) ;
d) l'éventuelle détermination d'au moins un paramètre d'interaction ;
e) la variation desdites conditions telles que définies à l'étape b) en faisant varier un paramètre de réaction, dans lequel ledit paramètre de réaction est choisi parmi le groupe comprenant la température, la vitesse de flux, le pH et la force ionique, et ;
f) la répétition, au moins une fois, des étapes c) à e).

**2.** Procédé selon la revendication 1, dans lequel ledit substrat poreux est un substrat en flux continu.

**3.** Procédé selon l'une des revendications 1-2, dans lequel ledit substrat poreux est un substrat d'oxyde métallique.

**4.** Procédé selon l'une des revendications 1-3, dans lequel ledit subtrat poreux est un substrat d'oxyde d'aluminium.

**5.** Procédé selon l'une des revendications 1-4, dans lequel ladite incubation est dynamique et dans lequel ladite incubation dynamique comprend la soumission dudit substrat poreux à au moins un cycle de flux avant et arrière, d'un échantillon à travers le substrat poreux, dans lequel ledit flux avant et arrière est établi par l'application d'une pression positive et négative en alternance.

**6.** Procédé selon l'une des revendications 1-5, dans lequel ledit paramètre d'interaction est choisi parmi le groupe comprenant la spécificité, l'affinité, la constante d'association, le paramètre d'absorption, le paramètre de distribution, le paramètre de métabolisme et le paramètre d'excrétion.

**7.** Procédé selon l'une des revendications 1 à 6, dans lequel ladite surveillance comprend les étapes :

a) de détection d'un signal, ledit signal résultant d'une interaction telle que définie dans l'étape b) de la revendication 1, et ;
b) d'enregistrement du niveau dudit signal tel que défini dans l'étape a), ledit niveau étant indicatif de la force d'interaction molécule cible / analyte.

**8.** Procédé selon l'une des revendications 1-7, dans lequel lesdites mesure et surveillance sont effectuées en temps réel.

**9.** Procédé selon l'une des revendications 1-8, dans lequel lesdites mesure et surveillance sont effectuées en temps semi-réel.

**10.** Procédé selon l'une des revendications 1-9, dans lequel lesdites mesure et surveillance sont fonction de la température.

**11.** Procédé selon l'une des revendications 1-10, dans lequel lesdites mesure et surveillance sont fonction de la vitesse de flux.

**12.** Procédé selon l'une des revendications 1-11, dans lequel ladite variation dudit paramètre de réaction est graduelle.

**13.** Procédé selon l'une des revendications 1-12, dans lequel lesdites molécules cibles sont sélectionnées parmi le groupe comprenant des récepteurs d'hormone, des peptides, des enzymes, des oligonucléotides, des acides nucléiques, des oligosaccharides, des protéines, des anticorps monoclonaux, des fragments d'anticorps, des haptènes et des aptamères.

**14.** Procédé selon l'une des revendications 1-13, dans lequel ledit analyte est sélectionné parmi le groupe comprenant des anticorps, des anticorps monoclonaux, des fragments d'anticorps, des antisérums, des polynucléotides, des acides nucléiques, des peptides, des sites de liaison d'enzyme, des polysaccharides, des cellules, des membranes cellulaires, et des organelles.

**15.** Procédé selon l'une des revendications 1-14, dans lequel ledit substrat poreux comprend une multiplicité de molécules cibles dans des régions prédéfinies sur ledit substrat poreux.

**16.** Procédé selon la revendication 15, dans lequel lesdites régions prédéfinies sont disposées dans l'espace pour former des micro-réseaux.

**17.** Procédé selon l'une des revendications 1-16, dans lequel ledit signal détectable est sélectionné parmi le groupe comprenant un indice de réfraction, une activité cellulaire, une émission de lumière incluant la fluorescence, un changement d'absorbance, un changement de fluorescence, une absorbance, un changement de couleur, un transfert d'énergie par résonance de fluorescence, une émission radioactive, un changement de pH, un changement de température, et un changement de masse.

**18.** Procédé selon l'une des revendications 1-17, dans lequel ledit signal détectable est un signal émis par la lumière.

**19.** Procédé selon la revendication 18, dans lequel ledit signal détectable est un signal fluorescent.

**20.** Procédé selon la revendication 19, dans lequel ledit signal fluorescent est émis par un analyte marqué, ledit analyte étant associé à une molécule cible.

**21.** Procédé selon l'une des revendications 1-20, dans lequel ledit analyte est présent dans ledit échantillon à une concentration de $5 \times 10^{-4}$ nM à 10 nM.

**22.** Procédé selon la revendication 21, dans lequel ladite concentration est de $5 \times 10^{-3}$ nM à 1 nM.

**23.** Procédé selon la revendication 22, dans lequel ladite concentration est de $5 \times 10^{-2}$ nM à $10^{-1}$ nM.

**24.** Procédé selon l'une des revendications 1-23, dans lequel un excès d'analyte non marqué est ajouté.

**25.** Procédé selon la revendication 24, dans lequel ledit excès d'analyte non marqué est ajouté après finalisation de la formation d'un complexe molécule cible immobilisée / analyte.

**26.** Utilisation d'un procédé selon l'une des revendications 1-25, pour étudier la cinétique de liaison analyte-molécule.

**27.** Utilisation d'un procédé selon l'une des revendications 1-25, pour mesurer les constantes de dissociation des paires de ligand-récepteur.

**28.** Utilisation d'un procédé selon l'une des revendications 1-25, pour cribler des banques de composés thérapeutiques.

**29.** Utilisation d'un procédé selon l'une des revendications 1-25, pour cribler des banques de protéines de diagnostique.

**30.** Utilisation d'un substrat poreux pour la préparation d'un kit de micro-réseau pour la mise en oeuvre d'un procédé selon l'une des revendications 1-25.

| PBS | C12 | PBS | C12 | PBS |
|-----|-----|-----|-----|-----|
| C1 | B5 | C1 | B5 | PBS |
| C4 | H2 | C4 | H2 | PBS |
| E7 | G11 | E7 | G11 | Flu-oligo |

FIGURE 1

**FIGURE 2**

**FIGURE 3**

**FIGURE 4**

**FIGURE 5**

**FIGURE 6**

**FIGURE 7**

**FIGURE 8**

FIGURE 9

EP 1 512 012 B1

|   | 1 | 2 | 3 | 4 | 5 | 6 | 7 | 8 |
|---|---|---|---|---|---|---|---|---|
| A | Ref | PBS | PBS | PBS | PBS | PBS | PBS | PBS |
| B | Bovine 200 | Goat 200 | Human 200 | Mouse 200 | Rabbit 200 | Rat 200 | PBS | PBS |
| C | Bovine 50 | Goat 50 | Human 50 | Mouse 50 | Rabbit 50 | Rat 50 | PBS | Ref |
| D | Bovine 25 | Goat 25 | Human 25 | Mouse 25 | Rabbit 25 | Rat 25 | PBS | PBS |
| E | Ref | PBS | PBS | PBS | PBS | PBS | PBS | PBS |
| F | Bovine 200 | Goat 200 | Human 200 | Mouse 200 | Rabbit 200 | Rat 200 | PBS | PBS |
| G | Bovine 50 | Goat 50 | Human 50 | Mouse 50 | Rabbit 50 | Rat 50 | PBS | Ref |
| H | Bovine 25 | Goat 25 | Human 25 | Mouse 25 | Rabbit 25 | Rat 25 | PBS | PBS |

**FIGURE 10**

**FIGURE 11**

**FIGURE 12**

**FIGURE 13**

FIGURE 14

**FIGURE 15**

FIGURE 16